# EUROPÄISCHE PATENTSCHRIFT

(11) **EP 3 184 072 B1**
(45) Veröffentlichungstag und Bekanntmachung des Hinweises auf die Patenterteilung: **25.09.2019**
(21) Anmeldenummer: 16206688.0
(22) Anmeldetag: 23.12.2016
(51) Int. Cl.: A61B 90/11, A61B 17/34, A61B 17/00, A61B 90/00

(54) **GELENKANORDNUNG, FÜHRUNGSVORRICHTUNG, HERSTELLUNG SOWIE VERWENDUNG EINER GELENKANORDNUNG**
JOINT ASSEMBLY, GUIDING DEVICE, PRODUCTION AND USE OF A JOINT ASSEMBLY
ENSEMBLE ARTICULÉ, DISPOSITIF DE GUIDAGE, PRODUCTION ET UTILISATION D'UN ENSEMBLE ARTICULÉ

(30) Priorität: 23.12.2015 DE 102015122802
(43) Veröffentlichungstag der Anmeldung: 28.06.2017
(73) Patentinhaber: Karl Storz SE & Co. KG, 78532 Tuttlingen (DE)
(72) Erfinder: Mayer-Ullmann, Gregor, 80799 München (DE); Hogervorst, Jordi, 72072 Tübingen (DE); Ehrhardt, André, 78532 Tuttlingen (DE)
(74) Vertreter: Witte, Weller & Partner Patentanwälte mbB

(56) Entgegenhaltungen:
- WO-A1-02/37452
- CN-A- 102 306 471
- DE-U1-202009 018 442
- GB-A- 2 503 422

## Beschreibung

Gegenstand der vorliegenden Offenbarung ist eine Gelenkanordnung für eine Mehr-Achs-Führungsvorrichtung für ein Instrument, insbesondere für ein medizinisches Instrument oder ein Simulationsinstrument. Die Offenbarung betrifft ferner eine Führungsvorrichtung mit einer derartigen Gelenkanordnung sowie die Herstellung einer Gelenkanordnung. Schließlich betrifft die Offenbarung auch vorteilhafte Verwendungen solcher Gelenkanordnungen.

Aus der WO 02/37452 A1 ist eine Halteeinrichtung für eine medizinische Simulations-vorrichtung bekannt, die mehrere Bewegungsfreiheitsgrade für ein Instrument bereitstellt, das einen länglichen Schaft aufweist, wobei die Halteeinrichtung eine kardanische Aufhängung aufweist, die ein um eine erste Schwenkachse und um eine zweite Schwenkachse drehbar aufgehängtes kugelförmiges Element umfasst, in dem der Schaft des Instruments teilweise aufgenommen ist.

Die genannte WO 02/37452 A1 bezieht sich explizit auf Vorrichtungen zur Simulation operativer Eingriffe, insbesondere minimal-invasiver Eingriffe. Daneben ist die Verwendung derartiger Halteeinrichtungen, die zur Aufnahme und Führung von Instrumenten ausgebildet sind, auch bei Assistenzsystemen für reale medizinische Eingriffe vorstellbar. So kann es etwa auch bei realen minimal-invasiven chirurgischen Eingriffen von Vorteil sein, wenn die Instrumente, insbesondere endoskopische Instrumente, definiert geführt und gelagert sind. Auf diese Weise kann der Handhabungsaufwand für den Operateur verringert werden. Grundsätzlich ist auch in der Telemedizin ein Anwendungsfeld zu sehen, also bei diagnostischen und/oder therapeutischen Vorgängen, bei denen etwa der Operateur nicht unmittelbar (räumlich) beim Patienten bzw. am Patienten agieren kann. Endoskopische Instrumente und Simulationsinstrumente umfassen üblicherweise einen länglichen Schaft, an dessen distalem Ende ein Werkzeug, eine Optik oder ähnliches aufgenommen sein kann.

Ein weiteres Anwendungsgebiet für derartige Halteeinrichtungen für Instrumente ist etwa im Rahmen der technischen Diagnose oder Qualitätssicherung gegeben. Demgemäß kann es sich bei den Instrumenten etwa um Inspektionsgeräte, Sonden, Taster, Kameras, Sensoren oder Ähnliches handeln. Daneben ist grundsätzlich auch eine Anwendbarkeit auf dem Gebiet der Fertigungstechnik und/oder der Materialbearbeitung gegeben. Demgemäß kann es sich bei den Instrumenten um Bearbeitungswerkzeuge und/oder Verarbeitungswerkzeuge handeln.

Die aus der WO 02/37452 A1 bekannte Halteeinrichtung stellt eine Führung für ein Instrument bereit, welche kardanisch gelagert ist. Schwenkachsen der kardanischen Lagerung sind mit Aktuatoren gekoppelt, die Momente erzeugen können. Auf diese Weise kann insbesondere bei Simulationsanwendungen ein sogenanntes Force-Feedback realisiert werden, also eine Kraftrückmeldung oder Kraftrückwirkung. Auf diese Weise kann etwa bei der Simulation operativer Eingriffe Gewebe simuliert werden, das durch das Instrument kontaktiert wird, welches der Operateur gerade handhabt. Auf diese Weise bekommt der Operateur bspw. eine Rückmeldung dahingehend, ob es sich um weiches Gewebe, hartes Gewebe oder gar um Knorpelgewebe oder Knochen handelt.

Es hat sich jedoch gezeigt, dass die Halteeinrichtung gemäß der WO 02/37452 A1 einen gewissen Fertigungsaufwand und Montageaufwand erforderlich macht. Dies kann die Verbreitung und Anwendung derartiger Halteeinrichtungen limitieren.

Aus der GB 2 503 422 A ist eine Gelenkanordnung für 3D-Druck Objekte bekannt, mit einem Kugelgehäuse, das ein Außengehäuse und eine Kugelgestalt aufweist, und mit einem Pfannengehäuse, das eine Pfannengestalt aufweist. Das Kugelgehäuse und das Pfannengehäuse können miteinander verschnappt werden. Die GB 2 503 422 A zeigt keine integrale Fertigung des Kugelgehäuses und des Pfannengehäuses.

Vor diesem Hintergrund liegt der Erfindung die Aufgabe zugrunde, eine alternative Mehr-Achs-Führungsvorrichtung für ein Instrument und insbesondere eine Gelenkanordnung für eine solche Führungsvorrichtung anzugeben, die in einfacher Weise herstellbar ist, wobei vorzugsweise sowohl der Fertigungsaufwand als auch der Montageaufwand reduziert werden können. Dies soll möglichst ohne Abstriche bei der gewünschten Funktionalität bewirkt werden können. Ferner sollen die Gelenkanordnung und die mit der Gelenkanordnung versehene Führungsvorrichtung besonders kompakt gestaltet werden können, um den Bauraumaufwand zu minimieren. Schließlich soll im Rahmen dieser Offenbarung ein vorteilhaftes Verfahren zur Herstellung einer Gelenkanordnung angegeben werden, die zumindest einige der oben genannten Aspekte verwirklicht. Daneben sollen vorteilhafte Verwendungen einer solchen Gelenkanordnung angegeben werden, bei denen sich die vorstehend genannten Aspekte positiv auswirken können.

Die Aufgabe wird gelöst durch die Merkmale der unabhängigen Patentansprüche. Vorteilhafte Weiterbildungen der Erfindung sind in den Unteransprüchen gekennzeichnet.

Die Gelenkanordnung betreffend wird diese Aufgabe gelöst durch eine Gelenkanordnung für eine Mehr-Achs-Führungsvorrichtung für ein Instrument, wobei die Gelenkanordnung eine Mehrzahl von konzentrisch zueinander ausgerichteten, zumindest abschnittsweise sphärisch gestalteten Führungselementen aufweist, die als Bestandteil einer kombinierten sphärischen Lagerung ausgebildet sind und sphärische Kontaktbereiche umfassen, wobei zumindest zwei der Führungselemente generativ und integral in konzentrischer Ausrichtung gefertigt sind, und wobei die zumindest zwei Führungselemente eine Relativverschwenkung erlauben.

Erfindungsgemäß kann nämlich die Herstellung der Gelenkanordnung deutlich vereinfacht werden, wenn die Führungselemente, die Gelenkglieder bilden, gemeinsam gefertigt werden, wobei die Elemente direkt bei der Fertigung ineinander angeordnet werden. Mit anderen Worten bedarf es keiner separaten Montage der Führungselemente. Der Begriff integrale Fertigung bezieht sich auf die Fertigung im Verbund bzw. im bereits gefügten Zustand. Vorzugsweise bilden die Führungselemente eine sphärische Lagerung mit zwei nichtparallelen Schwenkachsen. Üblicherweise sind die beiden Schwenkachsen um 90° zueinander geneigt. Auf diese Weise kann die Gelenkanordnung die Funktion einer kardanischen Lagerung bereitstellen, etwa gemäß der oben genannten WO 02/37452 A1. Dies kann jedoch mit bedeutend geringerem Teileaufwand, Fertigungsaufwand und Montageaufwand erfolgen.

Die Führungselemente können als Kugelelemente oder Kugelabschnittselemente bezeichnet werden. Demgemäß sind die Führungselemente in zumindest einigen Ausgestaltungen als zumindest abschnittsweise kugelartig gestaltete Kugelelemente ausgeführt. Dies kann vollständig oder nahezu vollständig ausgeformte Kugeln und/oder Kugelschalen umfassen. Es sind jedoch auch ringartige, ringabschnittsartige, segmentartige oder segmentabschnittsartige Gestaltungen denkbar. Da weitere Elemente an zumindest einem der den Führungselemente aufgenommen sind, weisen die Kugelelemente zumindest teilweise Führungsausnehmungen und dgl. auf.

Die integrale Gestaltung führt dazu, dass mit nur einem Fertigungsvorgang schlussendlich zumindest zwei Teile gefertigt werden können, die relativ zueinander beweglich sind, insbesondere relativ zueinander verschwenkbar. Vorzugsweise sind die Führungselemente relativ zueinander jeweils um eine von zwei einander im Zentrum der Gelenkanordnung schneidenden Schwenkachsen (um einen bestimmten Betrag) verschwenkbar. Somit kann im Idealfall eine einzige integral gefertigte (Verbund-Komponente die Gelenkanordnung der Mehr-Achs-Führungsvorrichtung ausbilden. Es versteht sich, dass weitere Elemente hinzugefügt werden können, etwa um drei oder mehr Bewegungsachsen (entsprechend insgesamt drei oder mehr Bewegungsfreiheitsgraden) bereitzustellen.

Beispielhaft werden Bewegungsachsen und Bewegungsfreiheitsgrade der Mehr-Achs-Führungsvorrichtung unter Verweis auf ein kartesisches Koordinatensystem (X-Y-Z) veranschaulicht. Gemäß zumindest einiger Ausgestaltungen erlaubt die Gelenkanordnung eine definierte Verschwenkung des Instruments um die X-Achse sowie um die Y-Achse, die senkrecht zueinander ausgerichtet sind. Somit sind zwei Bewegungsachsen bzw. zwei Bewegungsfreiheitsgrade vorhanden. Eine Erweiterung der Mehr-Achs-Führungsvorrichtung kann durch eine Verschwenkbarkeit oder Verdrehbarkeit des Instruments um die Z-Achse bewerkstelligt werden, die senkrecht zur X-Achse und senkrecht zur Y-Achse ist. Auf diese Weise werden drei Bewegungsachsen bzw. drei Bewegungsfreiheitsgrade bereitgestellt.

Eine Erweiterung der Mehr-Achs-Führungsvorrichtung kann etwa durch eine translatorische Bewegbarkeit des Instruments entlang der Z-Achse bewerkstelligt werden. Auf diese Weise können insgesamt vier Bewegungsachsen bzw. vier Bewegungsfreiheitsgrade verwirklicht werden. Bei den vier Bewegungsachsen handelt es sich um drei Schwenkachsen bzw. Rotationsachsen (um die X-Achse, Y-Achse, Z-Achse) sowie um eine translatorische Achse für geradlinige Schubbewegungen (Bewegung entlang der Z-Achse).

Die zumindest zwei Führungselemente der Gelenkanordnung sind integral und generativ aus einem Werkstoff gefertigt, der in einem generativen Fertigungsverfahren verarbeitbar ist. Weiter bevorzugt trifft dies insgesamt auf drei konzentrisch ineinander angeordnete Führungselemente zu.

Zur generativen Fertigung eignen sich Kunststoffwerkstoffe, Metallwerkstoffe, ferner jedoch auch Keramikwerkstoffe und Verbundwerkstoffe. Ein beispielhaftes generatives Fertigungsverfahren ist der sogenannte 3D-Druck. Üblicherweise werden beim 3D-Druck Kunststoffwerkstoffe verarbeitet, auch die Verarbeitung von Metallwerkstoffen ist denkbar. Es sind jedoch auch andere generative Verfahren bekannt, wie etwa die Stereolithografie, selektives Lasersintern, selektives Laserschmelzen, usw. Es sind generative Fertigungsverfahren denkbar, die auch die Bearbeitung verhältnismäßig harter und/oder steifer Werkstoffe erlauben, wie etwa von Metallwerkstoffen, Keramikwerkstoffen oder dergleichen. Generative Fertigungsverfahren können einstufig durchführbar sein, wie etwa beim 3D-Druck, wo das zu erzeugende Gebilde direkt gedruckt wird. Es sind jedoch auch mehrstufige generative Fertigungsverfahren denkbar, bei denen beispielsweise ein Rohling durch einen generativen Verfahrensschritt erzeugt wird, der anschließend einem weiteren Verfahrensschritt unterzogen wird, wie etwa einem thermischen Bearbeitungsschritt. Es ist vorstellbar, die Führungselemente generativ aus einem Basiswerkstoff zu erzeugen, in den ein Zusatzwerkstoff injiziert wird.

Durch generative Fertigungsverfahren lassen sich zwei oder mehr konzentrisch ineinander angeordnete Führungselemente erzeugen, die in einfacher Weise eine mehrachsige sphärische Lagerung bereitstellen, welche mittels üblicher Fertigungstechnologien nur mit sehr hohem Fertigungsaufwand und Montageaufwand herstellbar ist.

Vorzugsweise kann die Gelenkanordnung durch das generative Fertigungsverfahren nachbearbeitungsfrei oder nachbearbeitungsarm hergestellt werden. Mittels generativer Fertigung können Bauteile erzeugt werden, deren Toleranzen die Nutzung als sphärische Lagerung in den bezeichneten Anwendungsfeldern gestatten. Zur weiteren Verbesserung der Oberflächenqualität bzw. der Führungsqualität ist es vorstellbar, die Bauteile gezielt unter Vorspannung zu setzen, so dass etwaige verbliebene Toleranzen minimiert werden können.

Gemäß einer weiteren Ausgestaltung der Gelenkanordnung ist an den zumindest zwei Führungselementen zumindest eine mit einer Mehrzahl von Erhebungen versehene Kontaktfläche ausgebildet, insbesondere eine genoppte Kontaktfläche. Diese Gestaltung kann deutlich zur Verringerung des Führungsspiels sowie zur Erhöhung der Genauigkeit beitragen.

Generative Fertigungsverfahren erlauben üblicherweise keine "Null-Toleranzen", da auf diese Weise schlussendlich massive Bauteile gefertigt werden würden. Somit werden ineinander verschachtelte Bauteile üblicherweise mit genügend "Luft" gefertigt, um die Trennung der Einzelteile zu gestatten. Gemäß dem oben genannten Aspekt werden beispielsweise noppenartige Erhebungen auf zumindest einer Kontaktfläche ausgebildet, so dass die Einzelteile (die Führungselemente) nach der generativen Fertigung sicher voneinander getrennt, also relativ zueinander verschwenkt werden können. Mit anderen Worten ist zwischen zwei benachbarten ineinander angeordneten Führungselementen zwar eine definierte Lücke ausgebildet, in dieser Lücke sind jedoch zumindest punktuell erhabene Elemente ausgebildet, die als Kontaktelemente zwischen den Führungselementen dienen.

So ist es vorstellbar, die zumindest zwei Führungselemente derart auszulegen, dass die Erhebungen, die einem Führungselement zugeordnet sind, Spitzen aufweisen, die gerade noch eine gegenüberliegende Kontaktfläche eines benachbarten Führungselements kontaktieren. Selbst dann, wenn es hierbei zu einer minimalen Durchdringung kommen sollte, kann diese eigentlich ungewünschte Verbindung gelöst werden, da die Führungselemente mit geringem Kraftaufwand relativ zueinander bewegt werden können.

Im fertigen Zustand, also bei der Verwendung als Gelenkanordnung, müssen die Führungselemente jeweils nur einen definierten Bereich für die Relativverschwenkung bereitstellen. Dies kann beispielsweise einen Schwenkbereich von ± 60°um die X-Achse sowie einen Schwenkbereich von ± 60°um die Y-Achse umfassen. Es ist also nicht erforderlich, dass die Führungselemente im Betrieb willkürlich relativ zueinander um große Drehwinkel oder Verschwenkwinkel rotierbar gestaltet sein müssen. Insbesondere ist es nicht erforderlich, dass Verschwenkwinkel > 180°um die X-Achse bzw. um die Y-Achse gewährleistet werden müssen. Dies kann wiederum genutzt werden, um die zumindest zwei Führungselemente in einer ersten Relativorientierung zueinander zu fertigen und durch eine definierte Relativbewegung in eine zweite Betriebsorientierung zu überführen. Dies kann wiederum genutzt werden, um an den Kontaktflächen, Erhebungen, Noppen oder dergleichen auszubilden, welche erst nach der definierten Relativbewegung in eine Orientierung bewegt werden, die ihrer Betriebsorientierung (inklusive der im Betrieb bereitgestellten Verschwenkwinkel) entspricht.

Gemäß zumindest einiger Ausgestaltungen sind die Führungselemente nicht mit ideal kugelförmig gestalteten Kontaktflächen versehen. Es können definierte Abweichungen von der idealen Kugelform vorgegeben werden, um etwa in der Fertigungsorientierung die Ausbildung der Erhebungen zu vereinfachen. In der Betriebsorientierung können die Erhebungen in engen Kontakt mit den diesen zugeordneten Gegenflächen gebracht werden. Auf diese Weise kann insgesamt das Führungsspiel noch weiter verringert werden.

Eine weitere Maßnahme zur Erhöhung der Genauigkeit kann eine definierte Vorspannung auf das äußere Führungselement betreffen, die von außen aufgebracht wird. Auf diese Weise können die Führungselemente zumindest abschnittsweise deformiert werden, um fertigungsbedingte Lücken zwischen den Führungselementen zu überwinden.

Insgesamt kann also auch bei der generativen Fertigung eine hohe Führungsgenauigkeit gewährleistet werden. Dies erlaubt die Verwendung der Gelenkanordnung auch in Einsatzgebieten bzw. bei Anwendungen, bei denen es auf hohe Präzision und Genauigkeit ankommt.

Gemäß einer weiteren Ausgestaltung der Gelenkanordnung umfasst diese drei Führungselemente, die ein Zentralelement, ein Zwischenelement und ein Außenelement ausbilden, die zueinander konzentrisch angeordnet und integral in konzentrischer Ausrichtung gefertigt sind. Mit anderen Worten sind die Führungselemente ineinander verschachtelt, vorzugsweise verliersicher bzw. lösesicher.

Die Verwendung dreier Führungselemente, die als Zentralelement, Zwischenelement und Außenelement ausgebildet sind, hat den Vorteil, dass jeweils zwischen dem Zentralelement und dem Zwischenelement sowie zwischen dem Zwischenelement und dem Außenelement eine der oben genannten Schwenkachsen (X-Achse, Y-Achse) definiert werden kann, wenn die Führungselemente dazu ausgebildet sind, jeweils eine entsprechende Schwenkbewegung lediglich um diese Achse zu erlauben.

Gemäß einer Weiterbildung der obigen Ausgestaltung ist das Außenelement als Kugelschale gestaltet, wobei das Außenelement das Zwischenelement verliersicher umschließt, und wobei das Zwischenelement als Kugelschale gestaltet ist und das Zentralelement verliersicher umschließt.

Gemäß einer weiteren Ausgestaltung weist das Zentralelement eine mittige, durchgehende Führungsöffnung auf, die zur Einführung eines Instrumentenschaftes oder eines Führungsschaftes ausgebildet ist. Vorzugsweise schneidet eine Längsachse des Schaftes des eingeführten Instruments das Zentrum der Gelenkanordnung, in dem sich auch die Schwenkachsen schneiden. Der Schnittpunkt, der sich im Zentrum der Gelenkanordnung befindet, dient gewissermaßen als Referenz für die erlaubten Bewegungen. Mit anderen Worten kann der Schnittpunkt auch als Nullpunkt des kartesischen Koordinatensystems verstanden werden. Es ist vorstellbar, das Zentralelement gemeinsam mit dem Führungsschaft integral zu fertigen. Es ist jedoch auch vorstellbar, das Zentralelement mit einem separat gefertigten Führungsschaft zu koppeln.

Gemäß einer weiteren Ausgestaltung der Gelenkanordnung weisen das Zwischenelement und das Außenelement nutartige Führungsausnehmungen auf, wobei zumindest eine Führungsausnehmung des Zwischenelements und zumindest eine Führungsausnehmung des Außenelements zueinander winklig versetzt und insbesondere um 90°zueinander versetzt ausgerichtet sind. Die n utartigen Führungsausnehmungen können auch als Durchbrüche in der Schalengestalt des Zwischenelements bzw. des Außenelements bezeichnet werden. Vorzugsweise durchragt der Führungsschaft bzw. durchragt das eingeführte Instrument sowohl die zumindest eine Führungsausnehmung des Zwischenelements als auch die zumindest eine Führungsausnehmung des Außenelements. Auf diese Weise trägt der Führungsschaft zur Definition der Relativlagen zwischen den Führungselementen bei.

Ferner ist es bevorzugt, wenn das Zwischenelement und das Außenelement jeweils zwei Führungsausnehmungen aufweisen, die an einander abgewandten (radialen) Enden der Kugelgestalt oder Kugelschalengestalt ausgebildet sind.

Gemeinsam definieren die Führungsausnehmungen des Zwischenelements und die Führungsausnehmungen des Außenelements eine Führungskulisse für den Führungsschaft bzw. den Schaft des eingeführten Instrumentes. Führungsausnehmungen des Zwischenelements definieren einen erlaubten Schwenkbereich um die Schwenkachse, der das Zwischenelement zugeordnet ist. Führungsausnehmungen des Außenelements definieren einen erlaubten Schwenkbereich bzw. Schwenkwinkel um die Schwenkachse, der das Außenelement zugeordnet ist. Somit können die Führungsausnehmungen des Zwischenelements und des Außenelements gemeinsam den gesamten erlaubten Schwenkbereich definieren (beispielsweise ± 60° um die X-Achse sowie ± 60° um die Y-Achse).

Gemäß einer Weiterbildung der oben genannten Ausgestaltung ist das Zentralelement mit einem Führungsschaft versehen oder zumindest koppelbar, der die nutartigen Führungsausnehmungen des Zwischenelements und des Außenelements zumindest teilweise durchragt. Der Führungsschaft erstreckt sich ausgehend vom Zentrum des Zentralelements radial nach außen. Der Führungsschaft und die Führungsöffnung des Zentralelements sind konzentrisch zueinander ausgerichtet.

Wenn das Zentrum der Gelenkanordnung als Referenz für das kartesische Koordinatensystem (X-Y-Z) betrachtet wird, definiert die Längserstreckung des Führungsschafts bzw. der Führungsausnehmung zumindest in einer Neutralposition die Z-Achse. Die Führungsöffnung erstreckt sich auch durch den Führungsschaft.

Gemäß einer weiteren Ausgestaltung der Gelenkanordnung ist am Außenelement ferner zumindest eine nutartige Durchführung für eine Übertragungsachse ausgebildet, die mit dem Zwischenelement koppelbar ist, wobei die Führungsausnehmung des Außenelements und die Durchführung zueinander winklig versetzt und insbesondere um 90°zueinander versetzt ausgerichtet sind. Durch die Durchführung im Außenelement ist die Zugänglichkeit des Zwischenelements für eine Schwenkachse zur Lagerung des Zwischenelements gewährleistet.

Zur Bereitstellung der definierten Verschwenkwinkel bzw. Verschwenkbereiche ist gemäß zumindest einiger Ausgestaltungen sowohl das Außenelement als auch das Zwischenelement an einem Basisgestell schwenkbar aufnehmbar. Somit können sowohl das Außenelement als auch das Zwischenelement nicht beliebig relativ zum Basisgestell und zueinander verschwenkt werden. Vielmehr ist etwa das Zwischenelement derart am Basisgestell gelagert, dass lediglich eine Verschwenkung des Zwischenelements um die X-Achse erlaubt ist. Demgemäß ist beispielhaft das Außenelement derart am Basisgestell aufgenommen, dass lediglich eine Verschwenkung um die Y-Achse erlaubt ist. Die Führungsausnehmungen des Außenelements und des Zwischenelements kreuzen einander und definieren gemeinsam eine Kulissenöffnung, durch die der Führungsschaft bzw. der Schaft des Instruments in das Zentralelement einführbar bzw. mit diesem koppelbar ist. Mit anderen Worten ist das etwa kugelförmig gestaltete Zentralelement als solches zwar grundsätzlich frei im Zwischenelement und somit auch (mittelbar) frei im Außenelement aufgenommen, die Schwenkorientierung des Zentralelements wird jedoch durch den Führungsschaft bzw. den Schaft des Instruments definiert, der die Führungsausnehmungen des Zwischenelements und des Außenelements durchragt.

Gemäß einer weiteren Ausgestaltung der Gelenkanordnung sind die zumindest zwei Führungselemente als Einwegbauteil gestaltet. Vorzugsweise sind gemäß dieser Ausgestaltung das Zentralelement, das Zwischenelement und das Außenelement als integral gefertigtes Einwegbauteil gestaltet. Die im Rahmen dieser Offenbarung skizzierten Fertigungsvereinfachungen schlagen sich in einem deutlich verringerten Herstellungsaufwand sowie Materialaufwand nieder. Dies erlaubt die Gestaltung als Einwegbauteil. Auf diese Weise ist die Gelenkanordnung ohne weiteres für hygienisch anspruchsvolle Anwendungen, etwa im medizinisch-operativen Bereich geeignet. Beispielhaft muss gemäß dieser Ausgestaltung keine Eignung zur Autoklavierbarkeit gegeben sein. Eine potenziell verschmutzte oder kontaminierte Gelenkanordnung kann in einfacher Weise ausgetauscht werden. Dies kann trotz der Gestaltung als Einwegbauteil insgesamt die Systemkosten für die Führungsvorrichtung, bezogen auf einen einzelnen Anwendungsfall, reduzieren.

Gemäß einer weiteren Ausgestaltung ist sowohl am Zwischenelement als auch am Außenelement jeweils zumindest eine Aufnahme für einen Lagerflansch ausgebildet. Der Lagerflansch ist dazu ausgebildet, das Zwischenelement bzw. das Außenelement schwenkbar mit dem Basisgestell zu koppeln. Es ist jedoch grundsätzlich auch vorstellbar, auch die Gelenklager zur Aufnahme des Zwischenelements und des Außenelements zumindest teilweise als integralen Bestandteil der Gelenkanordnung auszubilden. Vorzugsweise sind die Aufnahmen des Außenelements und des Zwischenelements senkrecht zueinander ausgerichtet.

Die Führungsvorrichtung betreffend wird die Aufgabe der Erfindung durch eine Mehr-Achs-Führungsvorrichtung für ein Instrument, insbesondere ein medizinisches Instrument oder ein Simulationsinstrument, gelöst, wobei die Führungsvorrichtung ein Basisgestell und eine Gelenkanordnung nach zumindest einem der vorstehend erläuterten Aspekte aufweist, die am Basisgestell aufgenommen ist, wobei die Gelenkanordnung zwei nichtparallele Schwenkachsen bereitstellt, die sich vorzugsweise in einem Zentrum der Gelenkanordnung schneiden. Aus Veranschaulichungsgründen werden die Schwenkachsen mit X und Y bezeichnet.

Auch auf diese Weise wird die Aufgabe der Erfindung vollständig gelöst. Die nichtparallelen Schwenkachsen können auch als sich schneidende Schwenkachsen, ferner jedoch auch als windschief angeordnete Schwenkachsen bezeichnet werden. Das Basisgestell kann allgemein als Basisgehäuse bzw. als Basisrahmen bezeichnet werden.

Gemäß einer Weiterbildung der Führungsvorrichtung weist diese ferner einen ersten Schwenkaktuator für die erste Schwenkachse X, der mit einem Zwischenelement der Gelenkanordnung gekoppelt ist, und einen zweiten Schwenkaktuator für die zweite Schwenkachse Y auf, der mit einem Außenelement der Gelenkanordnung gekoppelt ist, wobei der erste Schwenkaktuator über eine Übertragungsachse mit dem Zwischenelement gekoppelt ist, die das Außenelement durchragt. Es versteht sich, dass die Zuordnung auch umgekehrt sein kann.

Auf diese Weise wird für jede der Schwenkachsen X, Y ein Antrieb bereitgestellt. Die Schwenkaktuatoren können beispielhaft als Force-Feedback-Antriebe (Kraft-Rückkopplungs-Antriebe) gestaltet sein. Auf diese Weise können etwa bei der Verwendung in einem Simulationssystem definierte Widerstände "simuliert" werden. Die Schwenkaktuatoren können jedoch auch bei der Verwendung als Assistenzsystem für chirurgische und/oder operative Eingriffe genutzt werden. Durch geeignete Ansteuerung der Schwenkaktuatoren kann dem Operateur direkt über das Instrument ein definiertes kraftbasiertes Signal ausgegeben werden. Auf diese Weisen kann beispielsweise ein Operationsgebiet (im Inneren eines menschlichen oder tierischen Körpers) in erlaubte Bereiche und nicht erlaubte Bereiche unterteilt werden. Sollte der Operateur irrtümlich versuchen, mit dem Instrument in einen nicht erlaubten Bereich einzudringen, kann ihm durch eine entsprechende Rückmeldung (erhöhter Widerstand) eindeutig signalisiert werden, dass er in einen unerlaubten Bereich einzudringen droht.

Die Schwenkaktuatoren können jedoch auch dazu ausgestaltet sein, das Instrument autark zu steuern. Anwendungen können sich beispielsweise im Bereich der Telemedizin, der (technischen und medizinischen) Diagnose sowie auch im Bereich der Simulation (zu technischen und/oder medizinischen Zwecken) ergeben.

Ferner ist es gemäß einer weiteren Ausgestaltung bevorzugt, wenn die Schwenkaktuatoren dazu ausgebildet sind, mittelbar oder unmittelbar eine aktuelle Schwenkposition in Bezug auf die diesen zugeordnete Schwenkachse (X oder Y) zu erfassen. Auf diese Weise kann eine Positionsüberwachung realisiert werden. Die Positionserfassung kann absolut und/oder relativ erfolgen.

Gemäß einer weiteren Ausgestaltung weist die Führungsvorrichtung ferner einen Führungsschaft auf, der an ein Zentralelement der Gelenkanordnung gekoppelt ist und eine Geradführung für das Instrument ausbildet. Somit wird dem Instrument bzw. dem Instrumentenschaft eine Translationsachse zur Verfügung gestellt. Die Translationsachse wird auch als Z-Achse bezeichnet. Auch die Z-Achse schneidet vorzugsweise das Zentrum der Gelenkanordnung.

Gemäß einer weiteren Ausgestaltung definieren der Führungsschaft und das Zentralelement ferner eine Rotationsachse Z für das Instrument. Somit kann das Instrument um die Z-Achse verdreht werden, die konzentrisch zur Längsachse des Instrumentenschafts ausgerichtet ist. Insgesamt kann die Führungsvorrichtung somit vier Achsen bzw. vier Bewegungsfreiheitsgrade für das Instrument bereitstellen.

Gemäß einer Weiterbildung der obigen Ausgestaltung weist die Führungsvorrichtung ferner einen Translationsaktuator auf, der dem Führungsschaft zugeordnet ist, und der dazu ausgebildet ist, zur translatorischen Kraftübertragung an den Instrumentenschaft anzugreifen, der in den Führungsschaft eingeführt ist. Zu diesem Zweck kann der Translationsaktuator mit einem Zahnrad versehen sein, das mit einer sich geradlinig erstreckenden Verzahnung des Instrumentenschafts zusammenwirkt. Mit anderen Worten kann der Instrumentenschaft zumindest abschnittsweise zahnstangenartig gestaltet sein.

Auch der Translationsaktuator kann als Force-Feedback-Aktuator gestaltet sein. Ferner kann der Translationsaktuator dazu ausgestaltet sein bzw. derart angesteuert werden, dass das Instrument selbsttätig durch den Translationsaktuator translatorisch entlang der Z-Richtung bewegt werden kann.

Gemäß einer weiteren Ausgestaltung weist die Führungsvorrichtung ferner einen Rotationsaktuator auf, der dem Führungsschaft zugeordnet ist, und der dazu ausgebildet ist, zur rotatorischen Kraftübertragung mittelbar oder unmittelbar an den Instrumentenschaft anzugreifen, der in den Führungsschaft eingeführt ist. Auf diese Weise kann auch die Rotationsbewegung um die Z-Achse eine Force-Feedback-Funktionalität aufweisen. Ferner kann der Rotationsaktuator dazu ausgestaltet sein, den Instrumentenschaft selbsttätig zu rotieren.

Gemäß einer Weiterbildung dieser Ausgestaltung wirkt der Rotationsaktuator mittelbar über den Führungsschaft auf den Instrumentenschaft ein. Dies heißt mit anderen Worten, der Rotationsaktuator kann beispielsweise ein Ritzel umfassen, das mit einem Zahnrad gekoppelt ist, das am Führungsschaft angeordnet ist. Das Ritzel kann das Zahnrad um die Z-Achse verdrehen. Insbesondere dann, wenn der Instrumentenschaft verdrehsicher im Führungsschaft aufgenommen ist, kann die Verdrehung um die Z-Achse mittelbar induziert werden. Andere Gestaltungen sind denkbar.

Gemäß einer weiteren Ausgestaltung der Führungsvorrichtung ist der Führungsschaft über eine Führungseinheit mit dem Basisgestell gekoppelt, wobei die Führungseinheit mit einer Verdrehsicherung gekoppelt ist, vorzugsweise mit einer Führungskette, die das Basisgestell und die Führungseinheit gelenkig miteinander verbindet. Vorzugsweise ist die Führungsvorrichtung an einem Ende des Führungsschaftes angeordnet, das vom Zentrum der Gelenkanordnung abgewandt ist. Wie vorstehend bereits erwähnt, kann es von Vorteil sein, wenn der Führungsschaft um die Z-Achse verdrehbar ist, um eine Rotationsachse für den Instrumentenschaft bereitzustellen. An dem Ende des Führungsschafts, das der Führungseinheit abgewandt ist, ist jedoch das Zentralelement der Gelenkanordnung ausgebildet. Insofern ist es von Vorteil, die Führungseinheit am abgewandten Ende anzuordnen.

Vorzugsweise wird eine Gestaltung bereitgestellt, bei der die Führungseinheit eine definierte Drehlage (in Bezug auf die Z-Achse) aufweist, da die Führungseinheit auch als Drehreferenz für den (drehbaren) Führungsschaft fungiert. Dabei muss jedoch beachtet werden, dass der Führungsschaft um die X-Achse bzw. die Y-Achse definiert verschwenkbar ist. Daher ist die Verdrehsicherung vorteilhaft dazu ausgebildet, die Drehlage der Führungseinheit hinreichend genau zu fixieren (in Bezug auf die Z-Achse), jedoch das gemeinsame Verschwenken der Führungseinheit mit dem Führungsschaft um die X-Achse bzw. um die Y-Achse zu erlauben. Dies wird vorteilhaft durch eine Führungseinheit in Form einer Führungskette bewirkt, die vorzugsweise gelenkig am Basisgestell und an der Führungseinheit aufgenommen ist.

Ein weiterer Vorteil der Führungskette besteht darin, dass die Führungskette auch als Schleppkette (Kabelschlepp) verwendet werden kann. Üblicherweise weist eine Schleppkette eine Mehrzahl von biegesteif gestalteten Kettengliedern auf, die über parallel zueinander ausgerichtete Bolzen miteinander verbunden sind, wobei die Glieder kastenartig gestaltet sind und einen Zwischenraum bereitstellen, durch den etwa Kabel oder dergleichen geführt werden können. Schleppketten sind üblicherweise biegesteif (gegen seitliche Durchbiegungen aufgrund eines Kraftangriffs parallel zu den Kettenbolzen) gestaltet. Demgemäß eignet sich eine nach Art einer Schleppkette gestaltete Führungskette als Verdrehsicherung.

Die Führungskette ist jedoch vorzugsweise dazu ausgebildet, Schwenkbewegungen der Gelenkanordnung um die X-Achse sowie um die Y-Achse, die sich auch auf die Orientierung des Führungsschaftes und der Führungseinheit auswirken, durch eine Ausgleichsbewegung mitmachen zu können. Beispielhaft ist die Führungskette über ein drehbar aufgenommenes Anschlussstück mit der Führungseinheit sowie, an ihrem Ende, das der Führungseinheit abgewandt ist, über ein drehbar aufgenommenes Anschlussstück mit dem Basisgestell gekoppelt. Die Drehbarkeit der Anschlussstücke ist in einem Aufführungsbeispiel als Drehbarkeit bzw. Verschwenkbarkeit um eine Achse zu verstehen, die senkrecht zu den (parallelen) Bolzenachsen ausgerichtet ist.

Vorzugsweise ist der Rotationsaktuator an der Führungseinheit aufgenommen und somit über die Verdrehsicherung mit dem Basisgestell gekoppelt. Auf diese Weise kann der Rotationsaktuator etwa über ein Ritzel auf ein Zahnrad einwirken, das am Führungsschaft angeordnet ist, um sich gemeinsam mit dem Führungsschaft relativ zum Rotationsaktuator verdrehen zu können.

Gemäß einer weiteren Ausgestaltung ist zwischen der Führungseinheit und dem Führungsschaft eine Kabeldurchführung ausgebildet, die einen Schleifringkontakt umfasst, um eine Kabelführung vom Basisgestell über die Führungskette und die Führungseinheit hin zum Führungsschaft zu bewirken, insbesondere zum Translationsaktuator des Führungsschafts. Gemäß zumindest einiger Ausgestaltungen ist der Führungsschaft gemeinsam mit dem an diesem aufgenommenen Translationsaktuator um die Z-Achse verdrehbar gelagert, dies schließt auch eine Relativverdrehung gegenüber der (verdrehgesicherten) Führungseinheit ein. Insofern erlaubt die Drehdurchführung auch bei beliebigen Rotationswinkeln eine Signalübertragung bzw. Energieübertragung.

Gemäß einer weiteren Ausgestaltung der Führungsvorrichtung weist diese ferner eine Sensoreinheit auf, die dem Führungsschaft zugeordnet ist, wobei die Sensoreinheit dazu ausgebildet ist, eine Translationsbewegung und eine Rotationsbewegung des eingeführten Instruments zu erfassen. Vorzugsweise ist die Sensoreinheit an der Führungseinheit aufgenommen und demgemäß verdrehsicher mit dem Basisgestell gekoppelt, so dass sich der Führungsschaft mit dem darin eingeführten Instrument relativ zur Sensoreinheit verdrehen kann. Vorzugsweise weist die Sensoreinheit einen mehrdimensional wirksamen Sensor oder zwei miteinander gekoppelte Sensoren auf, die gemeinsam zur Erfassung der Translationsbewegung und der Rotationsbewegung ausgebildet sind.

Mit anderen Worten ist die Sensoreinheit dazu ausgebildet, Rotationen um die Z-Achse und Längsbewegung entlang der Z-Achse zu erfassen. Die Sensoreinheit überwacht die Bewegung des Instrumentenschafts. Die Sensoreinheit kann auch als 2D-Sensor bezeichnet werden. Die Rotation sowie die Längsbewegung kann mittelbar oder unmittelbar erfasst werden. Verschiedene Messprinzipien sind denkbar, etwa optische Sensoren, Lasersensoren, induktive Sensoren, kapazitive Sensoren, und dergleichen. Ferner kann die Sensoreinheit grundsätzlich absolut messende Sensoren, alternativ oder zusätzlich jedoch auch relativ messende Sensoren umfassen.

Vorzugsweise weist die Führungsvorrichtung insgesamt eine Sensoreinrichtung auf, der die Sensoreinheit zugeordnet ist, wobei die Sensoreinrichtung dazu ausgebildet ist, Schwenkbewegungen um die X-Achse, Schwenkbewegungen um die Y-Achse, Translationsbewegungen entlang der Z-Achse sowie Rotationsbewegungen um die Z-Achse zu erfassen. Auf diese Weise kann die Bewegung des Instruments, insbesondere des Instrumentenschafts, vollständig überwacht werden. Demgemäß kann auch eine Ist-Position einer Spitze des Instruments ermittelt werden. Auf diese Weise ist die Führungsvorrichtung vorzugsweise auch zur Lageüberwachung bzw. Lagedetektion ausgestaltet. Dies kann insbesondere im medizinisch-operativen Betrieb von Vorteil sein, da auf diese Weise aufwendige optische und/oder radiologische Verfahren zur Bestimmung der Position und/oder Orientierung des Instruments vermieden werden können. Die Fähigkeit zur Positionsbestimmung bzw. Positionsüberwachung führt auch zu Vorteilen bzw. zu einem vereinfachten Aufbau bei Simulationssystemen, da der Aufwand für zusätzliche Sensorik vermieden werden kann.

Das Verfahren betreffend wird die Aufgabe der Erfindung durch ein Verfahren zur Herstellung einer Gelenkanordnung für eine Mehr-Achs-Führungsvorrichtung für ein Instrument gelöst, wobei das Verfahren die folgenden Schritte umfasst:
- generative Fertigung einer Mehrzahl von zumindest abschnittsweise kugelartig gestalteten Führungselementen mit sphärischen Kontaktbereichen zur Erzeugung einer kombinierten sphärischen Lagerung,
wobei zumindest zwei der Führungselemente integral in konzentrischer Ausrichtung gefertigt werden, und wobei die zumindest zwei Führungselemente eine Relativverschwenkung erlauben.

Vorzugsweise umfasst das Verfahren ferner ein Erzeugen zumindest einer mit einer Mehrzahl von Erhebungen versehenen Kontaktflächen, insbesondere einer genoppten Kontaktfläche, in den Kontaktbereichen. Das Verfahren ist als generatives Verfahren ausgestaltet, beispielsweise als 3D-Druckverfahren, Stereolithografie-Verfahren, Lasersinterverfahren, Laserschmelzverfahren oder dergleichen. Das Verfahren kann vorzugsweise nachbearbeitungsarm oder nachbearbeitungsfrei durchgeführt werden.

Das Verfahren eignet sich zur Herstellung einer Gelenkanordnung gemäß zumindest einem der vorstehend beschriebenen Aspekte. Es versteht sich, dass das Verfahren gemäß zumindest einem der hierin beschriebenen Aspekte, die Ausgestaltungen der Gelenkanordnung beschreiben, weitergebildet sein kann.

Gemäß einer Weiterbildung des Verfahrens umfasst dieses ferner Folgendes:
- generative, integrale Fertigung eines Zentralelements, eines Zwischenelements und eines Außenelements, die drei Führungselemente bilden, die zueinander konzentrisch angeordnet sind,
wobei das Außenelement als Kugelschale gestaltet wird und das Zwischenelement verliersicher umschließt, wobei das Zwischenelement als Kugelschale gestaltet wird und das Zentralelement verliersicher umschließt, und wobei das Zentralelement eine mittige, durchgehende Führungsöffnung aufweist, die zur Einführung eines Instrumentenschaftes oder eines Führungsschaftes ausgebildet ist.

Beispielhaft umfasst das Verfahren ferner die Ausbildung nutartiger Führungsausnehmungen beim Zwischenelement und beim Außenelement, die während der generativen Fertigung eine erste Relativorientierung aufweisen, wobei nach der generativen Fertigung eine Relativbewegung zwischen dem Zwischenelement und dem Außenelement bewirkt wird, um eine zweite Relativorientierung zwischen den nutartigen Führungsausnehmungen zu erzielen, die einer Relativorientierung im Betrieb entspricht. Mit anderen Worten kann das Überführen der Führungselemente aus der Fertigungsorientierung in die Betriebsorientierung eine definierte Relativverschwenkung umfassen.

Die Verwendung betreffend wird die Aufgabe der Erfindung durch die Verwendung einer Gelenkanordnung, die gemäß einem der vorstehend genannten Aspekte ausgestaltet oder gemäß einem der vorstehend beschriebenen Verfahrensaspekte gefertigt ist, als sphärische Lagerung bei einer Instrumentenhalterung, insbesondere einer Instrumentenhalterung für ein Simulationssystem oder ein Assistenzsystem für minimal-invasive Eingriffe gelöst. Minimal-invasive Eingriffe umfassen die minimal-invasive Chirurgie sowie die minimal-invasive Diagnose. Es versteht sich, dass auch andere Anwendungen denkbar sind, etwa die Verwendung der Gelenkanordnung in einer Führungsvorrichtung zur technischen Diagnose oder Inspektion. Ferner ist die Verwendung bei (technischen) Simulationssystemen vorstellbar. Allgemein eignet sich die Gelenkanordnung zur kostengünstigen Realisierung einer mehrachsigen sphärischen Lagerung mit definierten Verschwenkwinkeln für ein Instrument mit einem stabförmigen Instrumentenschaft, welches zumindest zwei, vorzugsweise drei oder vier Freiheitsgrade aufweist.

Die Verwendung bei einer Instrumentenhalterung für ein Assistenzsystem für minimal-invasive Eingriffe erstreckt sich vorzugsweise auf minimal-invasive Eingriffe am menschlichen oder tierischen Körper.

Weitere Merkmale und Vorteile der Erfindung ergeben sich aus der nachfolgenden Beschreibung mehrerer bevorzugter Ausführungsbeispiele unter Bezugnahme auf die Zeichnungen. Es zeigen:
- Fig. 1: eine perspektivische Ansicht einer mit einer Führungsvorrichtung versehenen Instrumentenhalterung, an der ein Instrument aufgenommen ist;
- Fig. 2: eine weitere Ansicht der Führungsvorrichtung gemäß Fig. 1 in einer der Darstellung gemäß Fig. 1 entsprechenden Orientierung, wobei aus Veranschaulichungsgründen Komponenten ausgeblendet sind;
- Figuren 3 bis 6: Teildarstellungen der Gestaltung gemäß Fig. 1 im Bereich einer Gelenkanordnung, wobei Fig. 3 und Fig. 4 Schwenklagen in Bezug auf eine Schwenkachse (Y-Achse) und Fig. 5 und Fig. 6 Schwenklagen in Bezug auf eine andere Schwenkachse (X-Achse) veranschaulichen;
- Fig. 7: eine perspektivische Teildarstellung einer Gelenkanordnung, die bei einer Anordnung gemäß Fig. 1 verwendbar ist;
- Fig. 8: eine Darstellung der Anordnung gemäß Fig. 7, wobei aus Veranschaulichungsgründen ein Außenelement der Gelenkanordnung ausgeblendet ist;
- Fig. 9: eine vereinfachte explodierte Ansicht der Ausgestaltung gemäß den Figuren 7 und 8, wobei ein Zwischenelement und ein Außenelement geschnitten dargestellt sind;
- Fig. 10: eine perspektivische Teilschnittdarstellung der Gestaltung gemäß Fig. 1 in einer ersten Orientierung zur Veranschaulichung eines Schwenkantriebs;
- Fig. 11: eine weitere perspektivische Teilschnittdarstellung der Anordnung gemäß Fig. 10 in einer abweichenden Orientierung zur Veranschaulichung eines weiteren Schwenkantriebs;
- Fig. 12: eine weitere Teilschnittdarstellung der Anordnung gemäß Fig. 1;
- Fig. 13: eine rückwärtige perspektivische Teildarstellung der Anordnung gemäß Fig. 1 zur Veranschaulichung eines Translationsantriebs;
- Fig. 14: eine Teildarstellung der Anordnung gemäß Fig. 13 in einer von Fig. 13 abweichenden Orientierung;
- Fig. 15: eine Darstellung der Anordnung gemäß Fig. 14 in einer von Fig. 14 abweichenden Orientierung;
- Fig. 16: eine explodierte Darstellung der Anordnung gemäß Fig. 15, wobei ein Sensorgehäuse geschnitten dargestellt ist;
- Fig. 17: eine teilweise geschnittene Teilansicht einer Anordnung gemäß Fig. 1 zur Veranschaulichung der Ausgestaltung einer Führungskette;
- Fig. 18: eine perspektivische Teildarstellung einer alternativen Ausgestaltung einer Gelenkanordnung, die bei einer Anordnung gemäß Fig. 1 verwendbar ist;
- Fig. 19: eine schematische Schnittansicht einer von der Gestaltung gemäß Fig. 1 abweichenden Ausgestaltung einer mit einer Führungsvorrichtung versehenen Instrumentenhalterung;
- Fig. 20: eine schematisch stark vereinfachte Teilschnittdarstellung einer Gelenkanordnung zur Veranschaulichung von Kontaktbereichen zwischen Führungselementen; und
- Fig. 21: eine schematische Blockdarstellung einer exemplarischen Ausgestaltung eines Verfahrens zur Herstellung einer Gelenkanordnung mit einem sphärischen Führungselement.

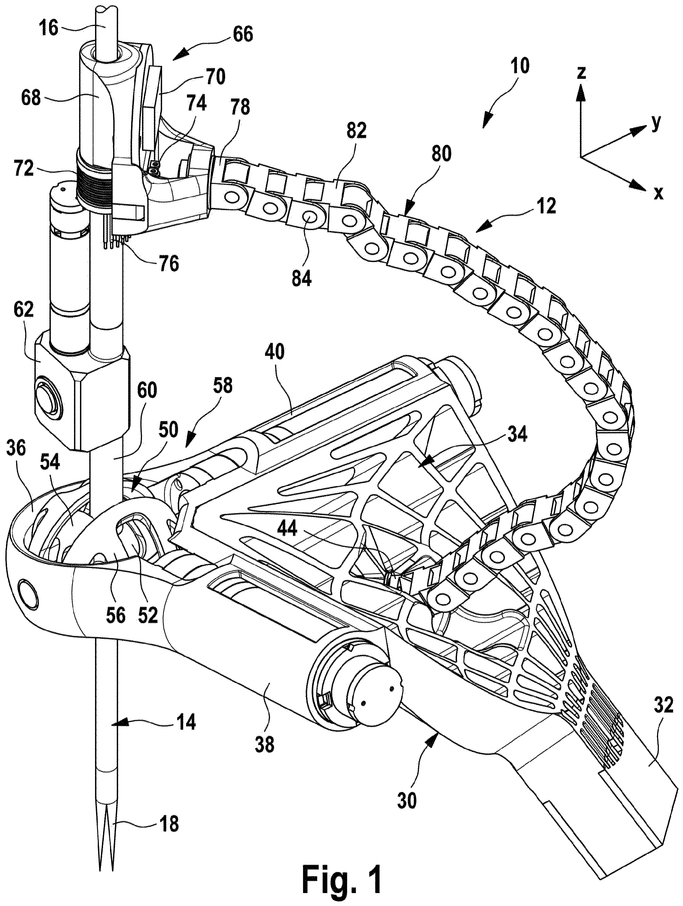

Fig. 1 zeigt eine perspektivische Ansicht einer Instrumentenhalterung, die insgesamt mit 10 bezeichnet ist. Die Instrumentenhalterung 10 weist eine Führungsvorrichtung 12 auf, die als Mehr-Achs-Führungsvorrichtung ausgestaltet ist. An der Führungsvorrichtung 12 ist ein Instrument 14 aufgenommen. Beispielhaft kann es sich bei dem Instrument 14 um ein Instrument zur minimal-invasiven Chirurgie oder um ein Simulationsinstrument für die minimal-invasive Chirurgie handeln. Ferner kann es sich bei dem Instrument 14 um ein Diagnoseinstrument, ein Inspektionsinstrument sowie um ein Simulationsinstrument für technische Anwendungen handeln, beispielsweise für die Bauteilinspektion, Fehlerdiagnose oder dergleichen.

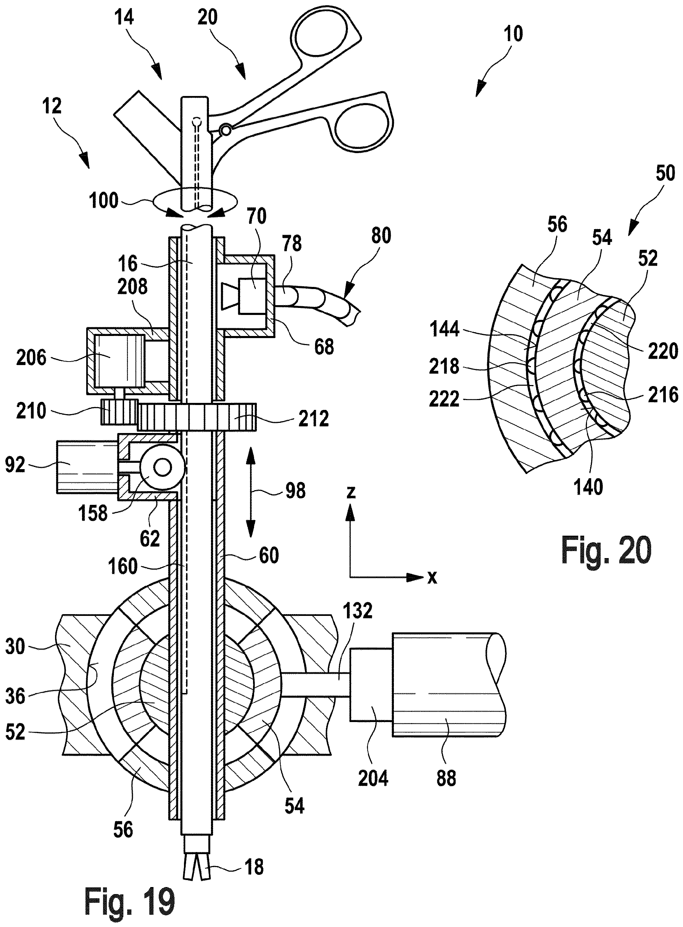

Das Instrument 14 weist einen Schaft 16 auf, der stabförmig ausgestaltet ist und dessen Längserstreckung deutlich größer als die Querschnittserstreckung ist. Das Instrument 14 ist über den Schaft 16 an der Führungsvorrichtung 12 aufgenommen. Üblicherweise weist der Schaft 16 ein distales und ein proximales Ende auf. In Fig. 1 ist am distalen Ende des Schafts 16 ein Werkzeug 18 angeordnet, beispielsweise ein Werkzeug zur minimal-invasiven Chirurgie. Zumindest gemäß einiger Ausführungsbeispiele ist am proximalen Ende des Schafts 16 eine Handhabe 20 angeordnet, vergleiche insbesondere Fig. 19. Über die Handhabe 20 kann das Werkzeug 18 angesteuert werden. Ferner kann die Position bzw. Orientierung des Instruments 14 insgesamt verändert werden, um mit dem Werkzeug 18 eine Zielposition erreichen zu können. Die Positionierbewegung erfolgt in definierter Weise, da das Instrument 14 an der Führungsvorrichtung 12 beweglich geführt aufgenommen ist.

Zu Erläuterungszwecken ist in zumindest einigen der nachfolgend beschriebenen Figuren ein (kartesisches) Koordinatensystem X-Y-Z dargestellt, das nachfolgend zur Veranschaulichung bestimmter Richtungen und Orientierungen benutzt werden soll. Es versteht sich, dass das Koordinatensystem X-Y-Z lediglich zur Veranschaulichung und Erläuterung dient und nicht dazu, den Gegenstand der Offenbarung einzuschränken. Es versteht sich ferner, dass zur Beschreibung der verschiedenen Ausgestaltungen und Aspekte dieser Offenbarung auch andere Koordinatensysteme mit anderen Orientierungen und Zuordnungen verwendbar sind. Es liegt im Bereich des fachmännischen Handelns, entsprechende (gedankliche) Transformationen vorzunehmen.

Dasselbe trifft nachfolgend auch auf Richtungsangaben und Angaben zur räumlichen Einordnung zu, wie etwa oben, unten, seitlich, vorne, hinten usw. Auch die Verwendung derartiger Begriffe soll nicht in einem einschränkenden Sinne verstanden werden. Sofern im Zusammenhang mit bestimmten Darstellungen und Orientierungen derartige Begriffe verwendet werden, können diese auf die tatsächlich gezeigte Darstellung bezogen sein und demgemäß bei abgewandelten Darstellungen, verbunden mit geänderten Orientierungen und Blickrichtungen, durch entsprechende abgewandelte Zuordnungsbegriffe ersetzt werden.

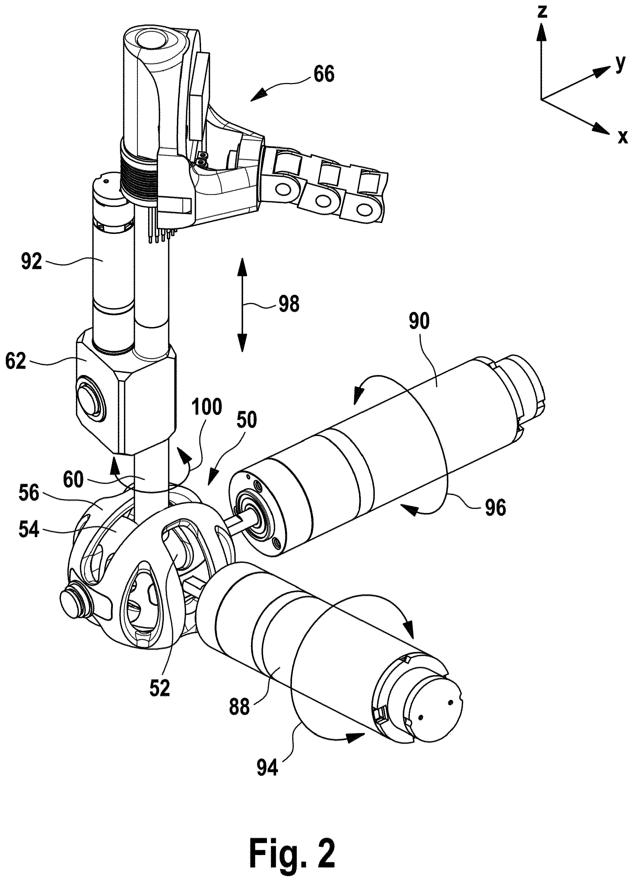

Zumindest in einer Neutralstellung ist der Schaft 16 des Instruments 14 parallel zur Z-Achse ausgerichtet. Die anhand der Fig. 1 veranschaulichte Führungsvorrichtung 12 ist als Mehr-Achs-Führungsvorrichtung ausgestaltet, insbesondere als 4-Achs-Führungsvorrichtung. Mit anderen Worten weist die Führungsvorrichtung 12 insgesamt vier Freiheitsgrade für das Instrument 14 auf. Diese Freiheitsgrade umfassen Schwenkbewegungen um die X-Achse sowie um die Y-Achse, eine translatorische Bewegung entlang der Z-Achse (bzw. entgegen der Z-Achse) sowie eine Rotation um die Z-Achse. Diese Bewegungsrichtungen werden beispielsweise in Fig. 2 durch Doppelpfeile 94, 96, 98, 100 veranschaulicht, auf die ergänzend Bezug genommen wird. Der Doppelpfeil 94 veranschaulicht eine Schwenkbewegung um die X-Achse. Der Doppelpfeil 96 veranschaulicht eine Schwenkbewegung um die Y-Achse. Der Doppelpfeil 98 veranschaulicht eine translatorische Schubbewegung entlang der Z-Achse. Der Doppelpfeil 100 veranschaulicht eine Rotationsbewegung um die Z-Achse.

Die Führungsvorrichtung 12 der Instrumentenhalterung 10 ist in besonderer Weise ausgestaltet, um die gewünschten Bewegungsfreiheitsgrade für das Instrument 14 bereitzustellen. Die Führungsvorrichtung 12 weist ein Basisgestell 30 auf, das auch als Gehäuse oder Rahmen bezeichnet werden kann. Das Basisgestell 30 weist in einem rückwärtigen Bereich ein Anschlussstück 32 auf, das auch als Adapterstück oder Anschlussflansch bezeichnet werden kann. Über das Anschlussstück 32 kann die Führungsvorrichtung gestellfest aufgenommen werden, etwa an einem Operationstisch oder an einer Vorrichtung zur Simulation von medizinischen Eingriffen. Bei der Verwendung im technischen Umfeld, etwa zu Inspektionszwecken oder Diagnosezwecken, kann das Basisgestell 30 über das Anschlussstück 32 etwa an einem Werkzeugtisch oder Ähnlichem aufgenommen werden.

Das Basisgestell 30 weist einen Zentralbereich 34 auf, der sich an das Anschlussstück 32 anschließt. Der Zentralbereich 34 weist beispielhaft eine versteifende Verrippung auf. An dem Ende des Zentralbereichs 34, das dem Anschlussstück 32 abgewandt ist, weist das Basisgestell 30 ein Gelenklager 36 auf, das auch als Pfanne bezeichnet werden kann. Ferner weist das Basisgestell 30 eine erste Antriebsaufnahme 38 und eine zweite Antriebsaufnahme 40 auf, die dem Gelenklager 36 und dem Zentralbereich 34 benachbart angeordnet sind. Mit anderen Worten bilden die erste Antriebsaufnahme 38 und die zweite Antriebsaufnahme 40 Flanken des Basisgestells 30, die in das Gelenklager 36 münden. Vorzugsweise ist die erste Antriebsaufnahme 38 etwa rechtwinklig zur zweiten Antriebsaufnahme 40 ausgerichtet. Ausgehend vom Anschlussstück 32 ist das Basisgestell 30 etwa auslegerartig oder pultdachartig gestaltet. Im Zentralbereich 34, etwa mittig, ist ein Lagerstück 44 angeordnet, über das das Basisgestell 30 gelenkig mit einer Führungskette 80 koppelbar ist.

Gemäß zumindest einigen Ausgestaltungen, die nicht Teil der Erfindung sind, ist das Basisgestell 30 einstückig integral gefertigt. Beispielhaft bietet sich zu diesem Zweck ein Spritzgussverfahren an. Insbesondere eine großzügige Verrippung im Zentralbereich 34 gewährleistet gleichwohl eine ausgezeichnete Steifigkeit sowie einen hinreichend hohen Formänderungswiderstand. Insbesondere dann, wenn nur eine kleine Losgröße des Basisgestells 30 zu fertigen ist, bieten sich zur Herstellung des Basisgestells 30 auch generative Fertigungsverfahren an.

Am pfannenartig ausgebildeten Gelenklager 36 des Basisgestells 30 ist eine Gelenkanordnung 50 definiert verschwenkbar aufgenommen. Zur Veranschaulichung einer beispielhaften Ausgestaltung der Gelenkanordnung 50 wird wiederum auf Fig. 1 und ergänzend auf Fig. 2 verwiesen. In Fig. 2 ist aus Veranschaulichungsgründen das Basisgestell 30 der Führungsvorrichtung 12 ausgeblendet.

Die Gelenkanordnung 50 ist als sphärische Gelenkanordnung ausgebildet. Die Gelenkanordnung 50 umfasst drei Führungselemente, die konzentrisch zueinander ausgerichtet und ineinander angeordnet sind. Im Zentrum der Gelenkanordnung 50 ist ein Zentralelement 52 aufgenommen, das kugelartig gestaltet ist. Die Führungselemente können auch als Kugelelemente bezeichnet werden. Das Zentralelement 52 wird von einem Zwischenelement 54 umschlossen, das kugelschalenartig gestaltet ist. Das Zwischenelement 54 wird von einem Außenelement 56 umschlossen, das kugelschalenartig gestaltet ist. Gemeinsam bilden das Zentralelement 52, das Zwischenelement 54 und das Außenelement 56 eine sphärische Lagerung 58. Die sphärische Lagerung 58 gestattet gemäß einer beispielhaften Ausgestaltung eine kardanische Lagerung des Instruments 14 an der Führungsvorrichtung 12.

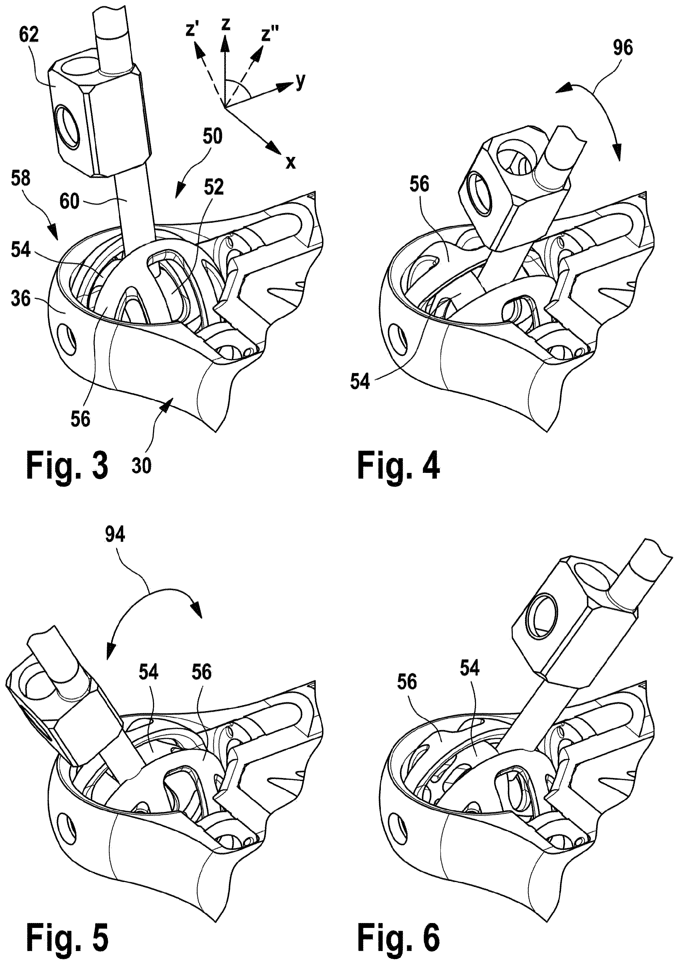

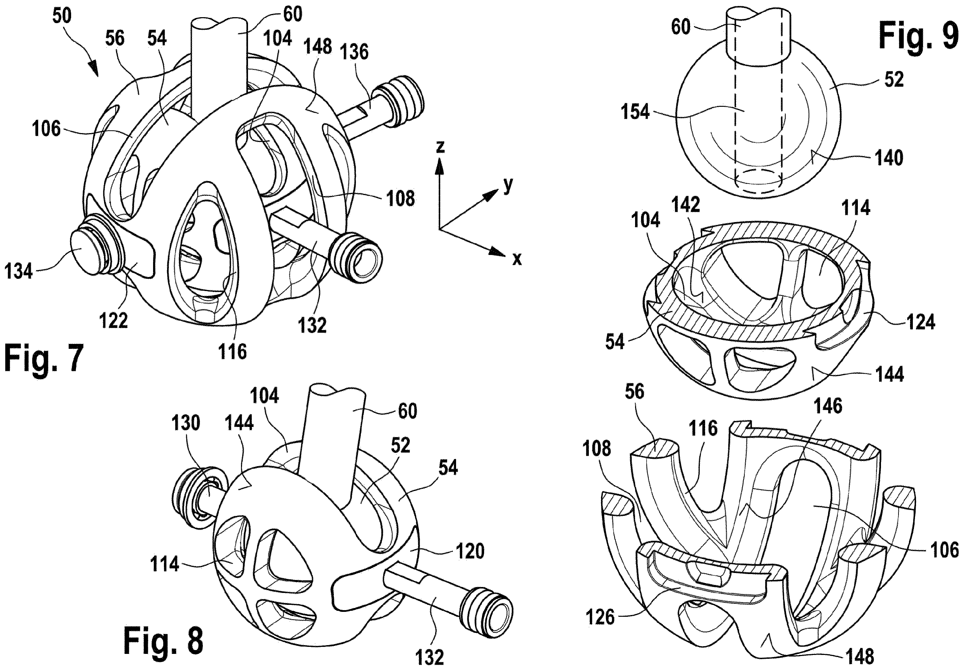

Zur Veranschaulichung der Bewegungsfreiheitsgrade, die die sphärische Lagerung 58 bereitstellt, wird ergänzend auf die Figuren 3 bis 6 verwiesen. Fig. 3 und Fig. 4 veranschaulichen eine Schwenkbewegung um die Y-Achse, vergleiche den Doppelpfeil 96. Fig. 5 und Fig. 6 veranschaulichen eine Schwenkbewegung um die X-Achse, vergleiche den Doppelpfeil 94. Es versteht sich, dass die sphärische Lagerung 58 auch Zwischenstellungen erlaubt, also auch gleichzeitige Auslenkungen um die X-Achse sowie um die Y-Achse. Detailansichten der Gelenkanordnung 50, die die sphärische Lagerung 58 ausbildet, können den Figuren 7, 8 und 9 entnommen werden, auf die später eingegangen wird. In Fig. 3 wird durch mit Z' und Z" angedeutete Achsrichtungen angedeutet, dass die Schwenkbewegung zu Abweichungen von der idealen Orientierung der Z-Achse führt.

Erneut wird Bezug auf Fig. 1 und Fig. 2 genommen. Der Gelenkanordnung 50 ist ferner ein Führungsschaft 60 zugeordnet, der am Zentralelement 52 aufgenommen ist, vergleiche auch Fig. 9. Der Führungsschaft 60 umschließt den Schaft 16 des Instruments 14 zumindest abschnittsweise. Der Führungsschaft 60 bestimmt eine aktuelle Orientierung des Instruments 16. Ein Nullpunkt des Koordinatensystems X-Y-Z ist beispielhaft im Zentrum der Gelenkanordnung 50 angeordnet. Setzt man dies voraus, so würde sich die Z-Achse relativ zur X-Achse bzw. zur Y-Achse verschwenken, also von ihrer senkrechten Orientierung abweichend angeordnet sein, wenn der Schaft 60 und das mit diesem gekoppelte Zentralelement um das Zentrum der Gelenkanordnung 50 verschwenkt werden. Der Einfachheit halber wird nachfolgend daran festgehalten, dass der Schaft 60 und das Zentralelement 52 die (momentane) Z-Achse für das Instrument 14 definieren. Am Führungsschaft 60 ist ferner eine dritte Antriebsaufnahme 62 angeordnet, die einen Translationsantrieb beherbergt.

Am proximalen Ende des Führungsschaft 60 ist eine Führungseinheit 66 angeordnet, die ein Sensorgehäuse 68 aufweist, das eine Sensoreinheit 70 beherbergt. Das Sensorgehäuse 68 ist als Verlängerung des Führungsschafts 60 ausgebildet. Zwischen dem Sensorgehäuse 68 und dem Führungsschaft 60 ist ferner ein Ringkontakt 72 angeordnet, der einer Drehdurchführung für Kabel zugehörig ist. Der Führungsschaft 60 ist um seine Längsachse (Z-Achse) verdrehbar. Die Führungseinheit 66 wird durch eine Verdrehsicherung an einer solchen Verdrehung um die Z-Achse gehindert. Demgemäß kann es zu einer Relativverdrehung zwischen der Führungseinheit 66 und dem Führungsschaft 60 kommen. Zur Übertragung von Signalen und von Energie von einem Anschluss 74, der der Führungseinheit 66 zugeordnet ist, zu Leitern 76, die dem Führungsschaft 60 zugeordnet sind, ist ein Drehübergang mit dem Ringkontakt 72 vorgesehen, der beispielsweise über Schleifkontakte kontaktiert wird.

Am Sensorgehäuse 68 der Führungseinheit 66 ist ferner ein Lagerstück 78 angeordnet, das zur gelenkigen Anbindung der Führungskette 80 ausgebildet ist. Die Führungskette 80 erstreckt sich somit zwischen dem Lagerstück 44 am Basisgestell 30 und dem Lagerstück 78 am Sensorgehäuse 68. Die Führungskette 80 umfasst eine Mehrzahl von Kettengliedern 82, die durch Bolzen 84 miteinander verbunden sind. Vorzugsweise ist die Führungskette 80 nach Art einer Schleppkette ausgestaltet und stellt einen Raum zur Kabelführung bereit. Mit anderen Worten kann die Führungskette 80 einen Kabelstrang beherbergen, der den Anschluss 74 kontaktiert. Die Führungskette 80 ist vorzugsweise als biegesteife Kette ausgestaltet und weist ein hohes Widerstandsmoment gegen seitlich einwirkende Kräfte auf, die parallel zu den Bolzen 84 orientiert sind. Mit anderen Worten ist die Führungskette 80 vorzugsweise hinreichend steif gestaltet, so dass die Bolzen 84 ihre parallele Ausrichtung zueinander behalten. Auf diese Weise wird sichergestellt, dass die Führungseinheit 66 keine oder nur eine geringe Relativverdrehung um die Z-Achse erfährt, wenn das Instrument 14 in der Führungsvorrichtung 12 bewegt wird. Die Führungskette 80, die über die Lagerstücke 78, 44 gelenkig am Basisgestell 30 sowie an der Führungseinheit 66 aufgenommen ist, ermöglicht eine gewünschte Verschwenkbarkeit der Führungseinheit um die X-Achse bzw. um die Y-Achse, gemeinsam mit dem Führungsschaft 60.

Die Sensoreinheit 70, die im Sensorgehäuse 68 aufgenommen ist, ist vorzugsweise als zweidimensional wirksame Sensoreinheit ausgestaltet. Mit anderen Worten ist die Sensoreinheit 70 dazu ausgebildet, eine Translationsbewegung des Instruments 14 entlang der (momentanen) Z-Achse sowie eine Verdrehung des Instruments 14 um die (momentane) Z-Achse zu erfassen, wobei die Verdrehung gemäß dem hier veranschaulichten Ausführungsbeispiel als gemeinsame Verdrehung des Führungsschafts und des Instruments 14 erfolgt. Mit anderen Worten ist das Instrument 14 verdrehsicher am Führungsschaft 60 aufgenommen, aber im Führungsschaft 60 translatorisch entlang der Z-Achse verfahrbar. Über die Führungskette 80 wird eine Rotationsreferenz bereitgestellt, da sich die Führungseinheit 66 nicht um die Z-Achse verdrehen kann.

Beispielhaft weist die Sensoreinheit 70 zwei Sensoren auf, von denen einer die Translationsbewegung und einer die Rotationsbewegung des Schafts 16 des Instruments 14 erfasst. Es ist jedoch auch vorstellbar, einen 2D-Sensor vorzusehen, etwa einen 2D-Lasersensor, der eine Oberfläche des Schafts 16 abtastet, um Translationsbewegungen und Rotationsbewegungen detektieren zu können.

Insbesondere der Ansicht gemäß Fig. 2 ist entnehmbar, dass die Führungsvorrichtung 12 einen ersten Antrieb 88, einen zweiten Antrieb 90 und einen dritten Antrieb 92 aufweist. Der erste Antrieb 88 ist der X-Achse zugeordnet. Der zweite Antrieb 90 ist der Y-Achse zugeordnet. Der dritte Antrieb 92 ist der Z-Achse zugeordnet. Die Antriebe 88, 90 sind als Schwenkantriebe ausgestaltet, vergleiche die Doppelpfeile 94, 96, die Schwenkbewegungen um die X-Achse bzw. die Y-Achse veranschaulichen. Der dritte Antrieb 92 ist als translatorischer Antrieb ausgestaltet, vergleiche den Doppelpfeil 100, der eine Verfahrbewegung entlang der Z-Achse veranschaulicht. Gemäß der anhand der Figuren 1 und 2 veranschaulichten Ausgestaltung umfasst die Führungsvorrichtung 12 keinen separaten Antrieb für die Rotationsbewegung um die Z-Achse, vergleiche den Doppelpfeil 100. Es sind jedoch andere Ausgestaltungen denkbar, bei denen ein entsprechender Antrieb vorgesehen ist, vergleiche hierzu auch Fig. 19, die später näher erläutert wird.

Die Antriebe 88, 90, 92 der Führungsvorrichtung 12 können beispielhaft als sogenannte Force-Feedback-Antriebe ausgestaltet sein (Kraftrückwirkungsantriebe). Auf diese Weise ist der Zweck der Antriebe 88, 90, 92 nicht primär, das Instrument 14, das an der Instrumentenhalterung 10 aufgenommen ist, aktiv zu bewegen, sondern den Benutzer beim Führen des Instruments 14 zu unterstützen. Grundsätzlich ist es jedoch zumindest für einige Anwendungen auch vorstellbar, das Instrument 14 vollautomatisiert über die Antriebe 88, 90, 92 zu bewegen, ohne dass es eines manuellen Eingriffs bedarf.

Ferner ist es vorstellbar, die Antriebe 88, 90, 92 mit entsprechender Sensorik zu koppeln, um eine Ist-Position bzw. eine Ist-Orientierung des Instruments 14 in Bezug auf die X-Achse, die Y-Achse und die Z-Achse zu ermitteln. So können etwa die Antriebe 88, 90 genutzt werden, um die Schwenkposition des mit den Zentralelement 52 gekoppelten Führungsschafts 60 indirekt über die momentanen Verschwenkpositionen des Zwischenelements 54 und des Außenelements 56 zu bestimmen. In ähnlicher Weise kann auch der dritte Antrieb 92 genutzt werden, um eine Längsposition des Instruments 14 entlang der Z-Achse zu bestimmen. Sofern jedoch die Führungsvorrichtung 12 mit der Sensoreinheit 70 versehen ist, die zur Erfassung der Translationslage des Instruments 14 ausgebildet ist, muss der dritte Antrieb 92 nicht zur Lagebestimmung herangezogen werden. Es versteht sich, dass zusätzlich oder alternativ zu den Antrieben 88, 90, 92 separate Sensoren verbaut sein können, die zur Positionsbestimmung nutzbar sind, etwa Drehwinkelsensoren, die mit dem ersten Antrieb 88 bzw. dem zweiten Antrieb 90 gekoppelt sind. Gleichwohl kann die Positionsbestimmung, insbesondere die Drehwinkelbestimmung auch indirekt erfolgen, wenn etwa der erste Antrieb 88 und der zweite Antrieb 90 als relative Drehwinkelgeber oder absolute Drehwinkelgeber genutzt werden.

Mit Bezug auf Fig. 7, Fig. 8 und Fig. 9 wird eine bespielhafte Ausgestaltung der Gelenkanordnung 50 näher erläutert. Die Gelenkanordnung 50 ist integral mittels generativer Fertigung hergestellt. Mit anderen Worten sind das Zentralelement 52, das Zwischenelement 54 und das Außenelement 56 gemeinsam gefertigt, wobei ferner das Zentralelement 52 verliersicher im Zwischenelement 54 angeordnet und das Zwischenelement 54 verliersicher im Außenelement 56 angeordnet wird. Mit anderen Worten können das Zentralelement 52, das Zwischenelement 54 und das Außenelement 56 nur voneinander getrennt werden, wenn zumindest zwei der Bauteile zerstört werden.

Der Explosionsansicht gemäß Fig. 9 ist entnehmbar, dass die Führungselemente 52, 54, 56 verschachtelt angeordnet sind. Ein gemeinsames Zentrum der Führungselemente 52, 54, 56 definiert vorzugsweise den Punkt, in dem sich die Schwenkachsen X, Y sowie die Translationsachse bzw. Rotationsachse Z schneiden. Auf diese Weise kann durch eine integral gefertigte Einheit, die die Führungselemente 52, 54, 56 ausbildet, die Funktion einer kardanischen Lagerung verwirklicht werden, die sonst nur mit hohem Fertigungsaufwand und Montageaufwand verwirklicht werden kann.

Das Zentralelement 52, das Zwischenelement 54 und das Außenelement 56 sind derart gestaltet, dass zwischen den einzelnen Elementen jeweils nur definierte Schwenkbewegungen gestattet sind. Am Zwischenelement 54 sind nutartige Führungsausnehmungen 104 ausgebildet. Am Außenelement 56 sind nutartige Führungsausnehmungen 106 ausgebildet. Die Führungsausnehmungen 104, 106 sind in einer Betriebskonfiguration der Gelenkanordnung 50 rechtwinklig zueinander orientiert, vergleiche auch Fig. 7. Ferner ist am Außenelement 56 zumindest eine nutartige Durchführung 108 ausgebildet, durch die das Zwischenelement 54 vom ersten Antrieb 88 kontaktiert werden kann, vergleiche hierzu auch Fig. 2.

Demgemäß sind das Zwischenelement 54 und das Außenelement 56 nicht als durchgängige Kugelschalen ausgestaltet, sondern als partiell unterbrochene Kugelschalen. Ferner kann es von Vorteil sein, Fenster, Ausbrüche oder Ähnliches beim Zwischenelement 54 bzw. beim Außenelement 56 vorzusehen. Auf diese Weise kann der Materialaufwand minimiert werden. Ferner können sich funktionale Vorteile ergeben, wie etwa eine bessere Anschmiegung der (verbliebenen) Kontaktflächen bzw. eine leichtgängige Bewegbarkeit. In Fig. 7 sind Fenster im Außenelement 56 mit 116 bezeichnet. In Fig. 8 sind Fenster im Zwischenelement 54 mit 114 bezeichnet.

Gemäß der in Fig. 7 gezeigten Betriebskonfiguration der Gelenkanordnung 50 durchragt der Führungsschaft 60 sowohl eine Führungsausnehmung 104 des Zwischenelements 54 als auch eine Führungsausnehmung 106 des Außenelements 56.

Am Zwischenelement 54 ist zumindest ein Lagerflansch 120 aufgenommen oder ausgebildet. Am Außenelement 56 ist zumindest ein Lagerflansch 122 aufgenommen oder ausgebildet. Gemäß anhand der Figuren 7 bis 9 veranschaulichten Ausgestaltung sind die Lagerflansche 120, 122, von denen jeweils zwei vorgesehen sind, als separate Teile ausgestaltet, die mit einem Grundkörper des Zwischenelements 54 bzw. des Außenelements 56 gefügt werden. Demgemäß weist das Zwischenelement 54 zumindest eine Aufnahme 124 für den zumindest einen Lagerflansch 120 auf. Ferner weist das Außenelement 56 zumindest eine Aufnahme 126 für den zumindest einen Lagerflansch 122 auf, vergleiche auch Fig. 9. Die Lagerflansche 120, 122 erlauben eine flächige Krafteinleitung in das Zwischenelement 54 bzw. das Außenelement 56 und können somit die Belastbarkeit der Gelenkanordnung 50 deutlich erhöhen.

Am Zwischenelement 54 ist ferner eine Lagerachse 130 sowie eine Übertragungsachse 132 aufgenommen, die gemeinsam die Schwenkachse für das Zwischenelement 54 (X-Achse) definieren. Die Lagerachse 130 und die Übertragungsachse 132 sind an den Lagerflanschen 120 aufgenommen. Die Lagerachse 130 ist im gefügten Zustand am Basisgestell 30 gelagert. Die Übertragungsachse 132 ist im gefügten Zustand mit dem ersten Antrieb 88 gekoppelt.

Am Außenelement 56 ist eine Lagerachse 134 sowie ein Übertragungsachse 136 aufgenommen, die mit den Lagerflanschen 122 gekoppelt sind. Im gefügten Zustand ist die Lagerachse 134 am Basisgestell 30 aufgenommen. Im gefügten Zustand ist die Übertragungsachse 136 mit dem zweiten Antrieb 90 gekoppelt. Gemeinsam definieren die Lagerachse 134 und die Übertragungsachse 136 eine Schwenkachse (Y-Achse) für das Außenelement 56.

Somit stellen das Zwischenelement 54 und das Außenelement 56 insgesamt zwei Schwenkachsen (X-Achse, Y-Achse) für den Führungsschaft 60 bereit, der am Zentral-element 52 aufgenommen bzw. mit diesem gekoppelt ist. Die Führungsausnehmungen 104, 106 definieren dabei erlaubte Verschwenkbereiche für den Führungsschaft 60.

Das Zentralelement 52 ist kugelartig gestaltet und in der pfannenartig gestalteten Aufnahme des Zwischenelements 54 gelagert. Das Zentralelement 52 und der daran aufgenommene Schaft 60 sind um die Längsachse des Schafts 60 (Z-Achse) grundsätzlich frei verdrehbar. Schwenkbewegungen um die X-Achse bzw. um die Y-Achse sind durch das Zusammenwirken mit dem Zwischenelement 54 und dem Außenelement 56 nur im erlaubten Bereich gestattet.

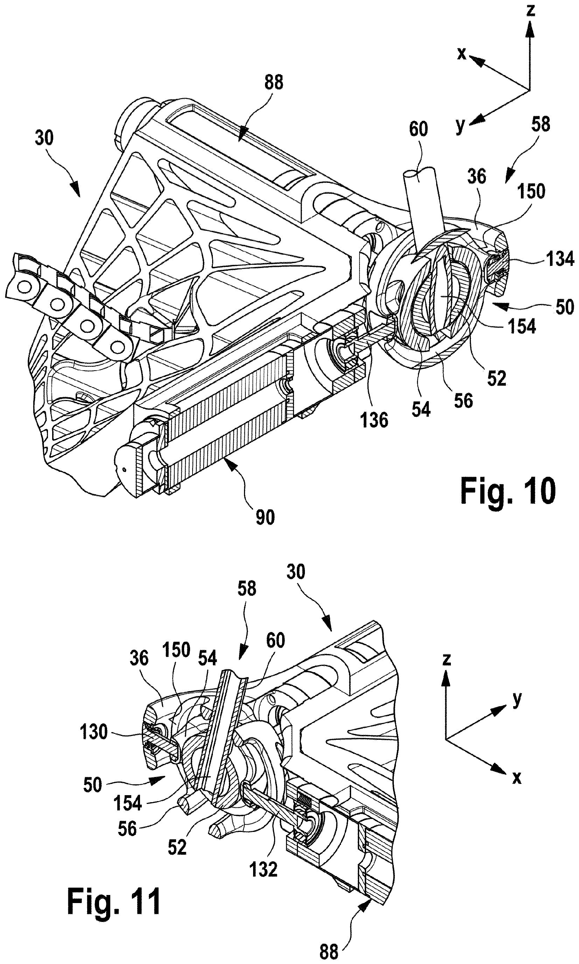

Am Zentralelement 52 ist eine sphärische Kontaktfläche 140 ausgebildet, die mit einer sphärischen Kontaktfläche 142 am Zwischenelement 54 zusammenwirkt. Die Kontaktfläche 140 ist als konvexe sphärische Kontaktfläche ausgestaltet. Die Kontaktfläche 142 ist als konkave sphärische Kontaktfläche ausgestaltet. Am Zwischenelement 54 ist eine äußere Kontaktfläche 144 ausgebildet, die mit einer inneren Kontaktfläche 146 des Außenelements 56 zusammenwirkt. Die Kontaktfläche 144 kann auch als konvexe sphärische Kontaktfläche bezeichnet werden. Die Kontaktfläche 146 kann auch als konkave sphärische Kontaktfläche bezeichnet werden. Ferner ist am Außenelement 56 eine äußere Kontaktfläche 148 ausgebildet. Die Kontaktfläche 148 kann auch als konvexe sphärische Kontaktfläche bezeichnet werden. Im gefügten Zustand, vergleiche etwa Fig. 1, kontaktiert die Kontaktfläche 148 eine Kontaktfläche 150, die am Gelenklager 36 des Basisgestells 30 zur Aufnahme und Führung der Gelenkanordnung 50 ausgebildet ist, vergleiche auch Fig. 10 und Fig. 11. Die Kontaktfläche 150 kann auch als konkave sphärische Kontaktfläche oder als Kontaktpfanne bezeichnet werden.

Fig. 9 veranschaulicht ferner, dass am Zentralelement 52 bzw. am Führungsschaft 60 eine sich durchgehend erstreckende Führungsöffnung 154 ausgebildet ist, die konzentrisch zur Z-Achse ausgerichtet ist. Im montierten Zustand durchragt der Schaft 16 des Instruments 14 die Führungsöffnung 154. Auch die Führungsöffnung 154 bzw. deren Längsachse schneidet das Zentrum der Gelenkanordnung 50.

Fig. 10 und Fig. 11 zeigen perspektivische Teilschnittansichten der Führungsvorrichtung 12, wobei die Schnittebene jeweils senkrecht zur durch die X-Achse und die Y-Achse aufgespannten Ebene orientiert ist. Fig. 10 zeigt einen Schnitt durch den zweiten Antrieb 90. Fig. 11 zeigt einen Schnitt durch den ersten Antrieb 88. Die Schnittebenen in den Figuren 10 und 11 sind um 90°zueinan der versetzt orientiert. Fig. 10 veranschaulicht, dass das Außenelement 56 der Gelenkanordnung 50 über die Lagerachse 134 am Basisgestell 30 aufgenommen ist. An der gegenüberliegenden Seite des Außenelements 56 ist diese über die Übertragungsachse 136 mit dem zweiten Antrieb 90 gekoppelt. Gleichermaßen veranschaulicht Fig. 11, dass das Zwischenelement 54 über die Lagerachse 130 am Basisgestell 30 aufgenommen ist. An der gegenüberliegenden Seite des Zwischenelements 54 ist dieses über die Übertragungsachse 132 mit dem ersten Antrieb 88 gekoppelt. Am Zentralelement 52 bzw. am Führungsschaft 60 stellt die Führungsöffnung 154 eine Aufnahme für den Schaft 16 des Instruments 14 bereit, die Achsen der beteiligten Komponenten schneiden sich im Zentrum der konzentrischen Gelenkanordnung 50.

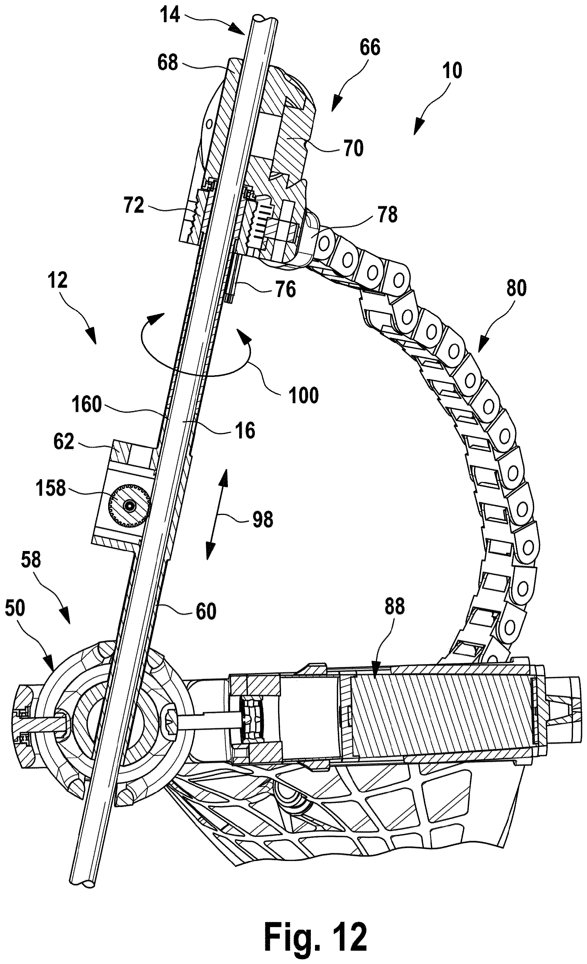

Fig. 12 veranschaulicht einen Längsschnitt durch den Führungsschaft 60, wobei die Schnittebene senkrecht zur Längsachse eines Zahnrads 158 gewählt ist, das in einem Gehäuse aufgenommen ist, das durch die dritte Antriebsaufnahme 62 bereitgestellt wird. Mit anderen Worten ist das Zahnrad 158 am Führungsschaft 60 aufgenommen und über den Führungsschaft 60 mit dem Zentralelement 52 der Gelenkanordnung gekoppelt. Das Zahnrad 158 greift an eine Verzahnung 160 des Schafts 16 des Instruments 14 an, wenn dieses in den Führungsschaft 60 eingeführt ist. Mit anderen Worten ist der Schaft 16 zumindest in einigen Ausgestaltungen abschnittsweise als Zahnstange ausgeführt bzw. mit einer zahnstangenartigen Verzahnung versehen.

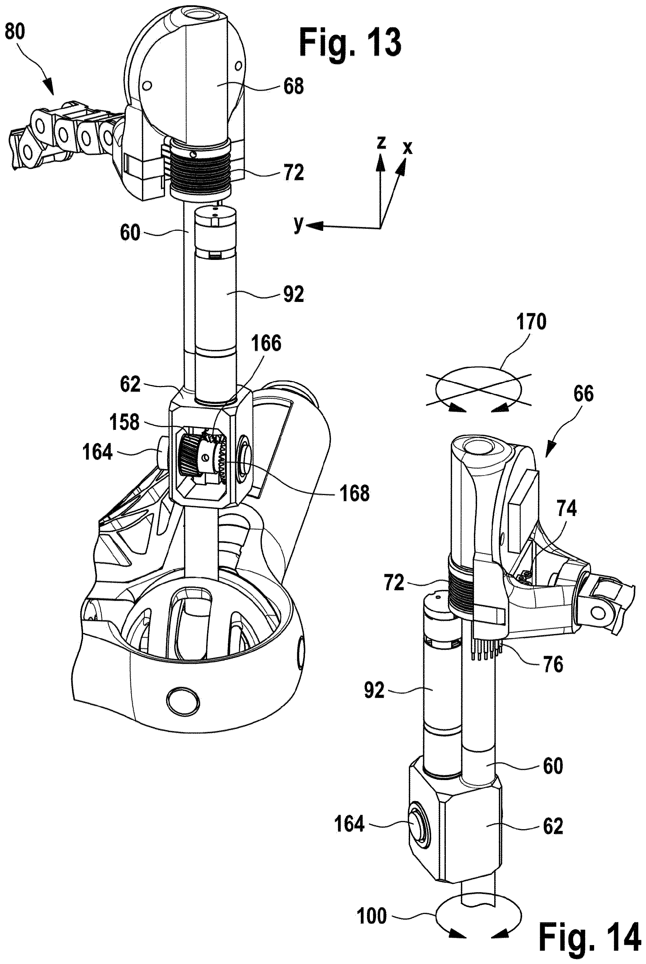

Ergänzend wird auf Fig. 13 verwiesen, die eine rückwärtige Ansicht auf das Gehäuse der dritten Antriebsaufnahme 62 zeigt. Das Zahnrad 158 ist auf einer Antriebswelle 164 angeordnet, welche senkrecht zur Längserstreckung des Führungsschafts 60 orientiert ist. Die Antriebswelle 164 ist ferner mit dem dritten Antrieb 92 gekoppelt, der senkrecht zur Antriebswelle 164 orientiert ist. Ein Abtrieb des dritten Antriebs 92 umfasst ein Ritzel 166, das kegelradartig gestaltet ist. Das Ritzel 166 greift an ein Tellerrad 168 an, das kegelradartig gestaltet ist. Gemeinsam erlauben das Ritzel 166 und das Tellerrad 168 eine 90°-Umlenkung der Antriebsbewegung des dritten Antriebs 92, so dass das Zahnrad 158 auf die Zahnstange 160 einwirkt, um das Instrument 14 translatorisch zu verfahren, vergleiche den Doppelpfeil 98 in Fig. 12.

Der dritte Antrieb 92 ist parallel zur Längserstreckung des Führungsschafts 60 orientiert. Auf diese Weise ist die Führungsvorrichtung 12 im Bereich des Führungsschafts 60 besonders kompakt gestaltet. Es versteht sich, dass der translatorische Antrieb für das Instrument 14 auch anderweitig gestaltet sein kann.

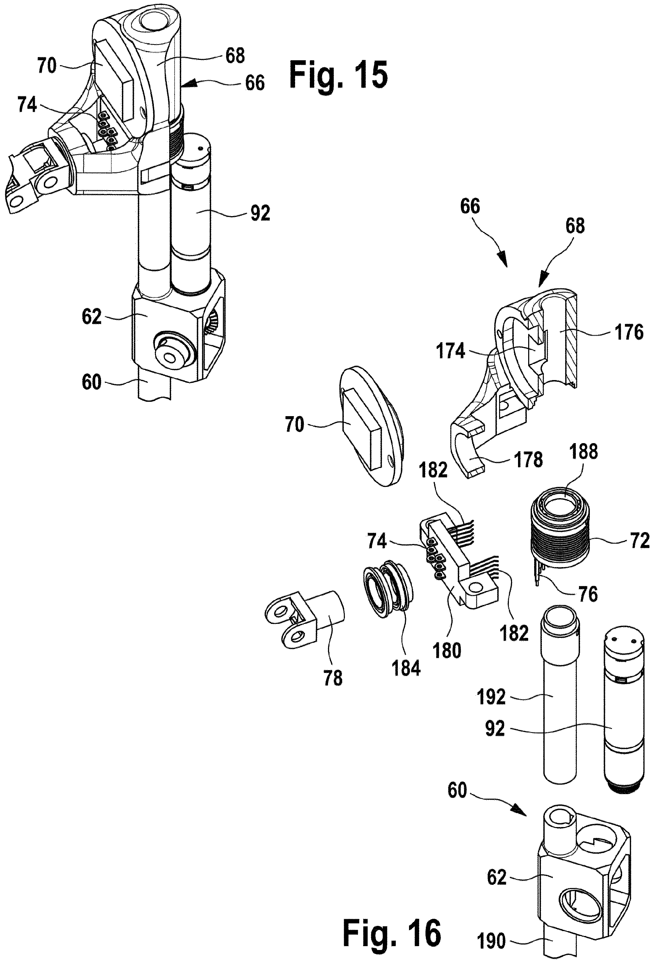

Mit erneutem Bezug auf Fig. 12 und ergänzendem Bezug auf die Figuren 13 bis 16 wird die Ausgestaltung der Führungseinheit 66 näher veranschaulicht. In Fig. 14 ist durch einen mit 170 bezeichneten durchkreuzten Pfeil angedeutet, dass die Führungseinheit 66 gerade nicht um die Z-Achse verschwenkbar gestaltet ist. Dies wird durch die Führungskette 80 bewirkt, die die Führungseinheit 66 am Basisgestell 30 gegen eine Verdrehung um die Z-Achse sichert. Um jedoch den dritten Antrieb 92 mit Energie sowie mit Steuersignalen zu versorgen, ist zwischen der Führungseinheit 66 und dem Führungsschaft 60 ein Drehübergang für die erforderlichen Versorgungsleitungen ausgebildet. Fig. 16 detailliert in einer explodierten Darstellung die Ausgestaltung der Führungseinheit 66 in einer Orientierung gemäß Fig. 15. Das Sensorgehäuse 68 weist eine Aussparung 174 für die Sensoreinheit 70 auf. Über die Aussparung 174 kann die Sensoreinheit 70 den Schaft 16 des Instruments 14 erfassen, der in einer Führung 176 des Sensorgehäuses 68 aufgenommen ist. Ferner ist am Sensorgehäuse 68 ein Lagersitz 178 ausgebildet, der etwa senkrecht zur Längserstreckung der Führung 176 orientiert ist. Am Lagersitz 178 ist das Lagerstück 78 unter Verwendung einer Lagerung 184 gelenkig aufgenommen.

Mit dem Sensorgehäuse 68 ist ferner ein Träger 180 gekoppelt, an dem die Anschlusskontakte 74 aufgenommen sind. Am Träger 180 sind ferner Schleifkontakte 182 ausgebildet, die im gefügten Zustand den Ringkontakt 72 kontaktieren. Der Ringkontakt 72 kann sich relativ zu den Schleifkontakten 182 verdrehen, ohne dass die Kontaktierung unterbrochen wird. Zwischen dem Ringkontakt 72 und dem Sensorgehäuse 68 ist ein Lager 188 ausgebildet, das eine Relativrotation zwischen dem Ringkontakt 72 (bzw. dem Führungsschaft 60) und dem Sensorgehäuse 68 der Führungseinheit 66 erlaubt. Der Ringkontakt 72 ist verdrehsicher am Führungsschaft 60 aufgenommen. Beispielhaft ist der Führungsschaft 60 mehrteilig gestaltet und mit einem unteren Schaftteil 190 und einem oberen Schaftteil 192 versehen. Der Ringkontakt 72 ist am oberen Schaftteil 192 verdrehsicher aufgenommen.

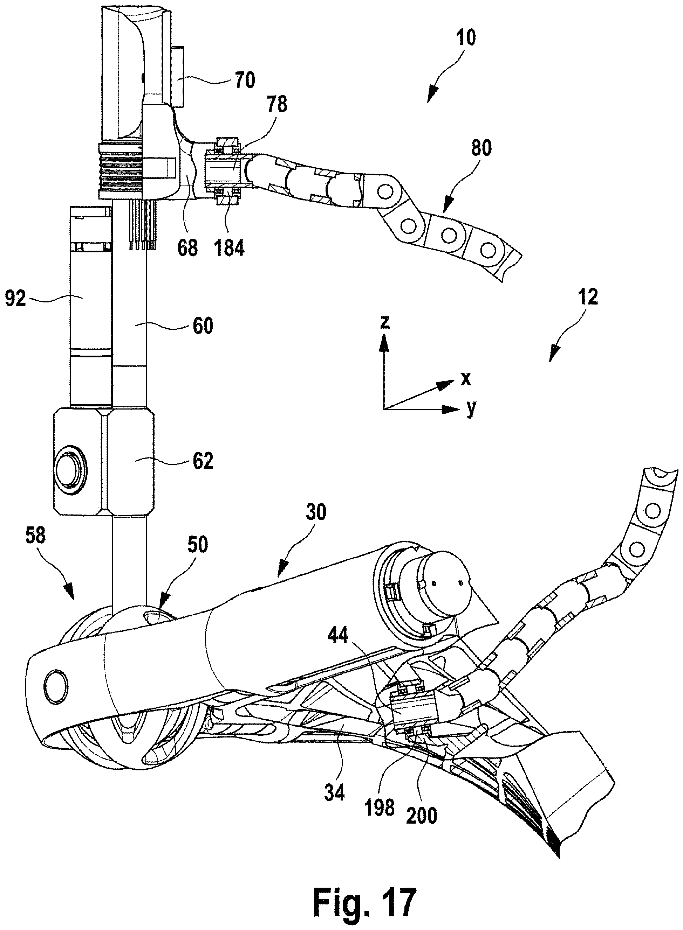

Fig. 17 zeigt eine teilweise geschnittene Ansicht der Instrumentenhalterung 10 gemäß Fig. 1, wobei die Ansichtsebene parallel zu der Ebene orientiert ist, in der sich die Führungskette 80 erstreckt. In Fig. 17 ist die Anbindung der Führungskette 80 über das Lagerstück 44 sowie das Lagerstück 78 geschnitten dargestellt. Das Lagerstück 44 ist am Basisgestell 30, insbesondere in dessen Zentralbereich 34, aufgenommen. Das Lagerstück 44 ist über eine Lagerung 198 gelenkig an einem Lagersitz 200 des Basisgestells 30 aufgenommen. Das Lagerstück 78 ist über die Lagerung 184 mit dem Lagersitz 178 des Sensorgehäuses 68 gekoppelt. Die als biegesteife Kette ausgestaltete Führungskette 80 gewährleistet, dass die Achsen der Lagerungen 184, 198 im Wesentlichen in der gleichen Ebene orientiert sind.

Demgemäß dient das Sensorgehäuse 68 auch als Referenz für die Drehorientierung des Führungsschafts 60 bzw. des Instruments 14 in Bezug auf die Z-Achse. Je nach aktueller Schwenkposition (Schwenkbewegung um die X-Achse bzw. um die Y-Achse) des Schafts 60 kann sich eine Neigung zwischen den Lagerungen 184, 198 verändern. Gleichwohl wird durch die Führungskette 80 sichergestellt, dass die Lagerungen 184, 198 die gemeinsame Ebene (bzw. einen definierten Toleranzbereich) nicht verlassen. Gemäß der in Fig. 17 gezeigten Ausgestaltung können sich der Führungsschaft 60, der am Zentralelement 52 aufgenommen ist und die dritte Antriebsaufnahme 62, der der dritte Antrieb 92 zugeordnet ist, frei um die Längsachse des Führungsschafts 60 verdrehen (die der Einfachheit halber als Z-Achse bezeichnet wird).

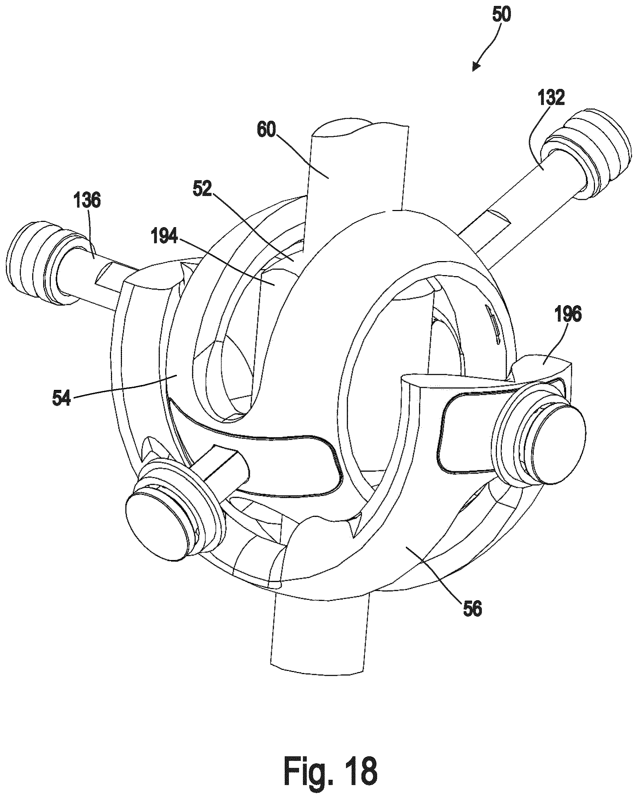

Fig. 18 veranschaulicht eine abgewandelte Ausführungsform einer Gelenkanordnung 50, die bei einer Führungsvorrichtung 12 einer Instrumentenhalterung 10 verwendbar ist. Im Hinblick auf die grundsätzliche kugelgelenkartige Gestaltung der Gelenkanordnung 50 wird ergänzend auf die Fig. 7 bis 9 verweisen.

In Fig. 18 sind beispielhaft das Zentralelement 52 und das Außenelement 56 als Kugelabschnitte bzw. Kugelschalenabschnitte gestaltet. Auch derartige Gestaltungen sind vom Umfang der vorliegenden Offenbarung abgedeckt. Das Zentralelement 52 weist eine Freisparung 194 auf, die die Kugelform beschneidet. Das Außenelement 56 weist eine Freisparung 196 auf, die die ringartige Gestaltung unterbricht. Gleichwohl sind auch das Zentralelement 52 und das Außenelement 56 in den Bereichen, die weitere sphärisch gestaltete Führungselemente kontaktieren können, mit spärischen Flächen oder Kugelabschnittsflächen versehen, um die Relativbewegung bzw. Relativverschwenkung der Führungselemente zu ermöglichen.

Die Gestaltung gemäß Fig. 18 kann eine Reduzierung des Fertigungsaufwandes, der Materialeinsatz und des Gewichts der Gelenkanordnung 50 zu Folge haben. Gleichwohl kann auch die Gelenkanordnung 50 gemäß Fig. 18 zumindest teilweise integral, insbesondere generativ, gefertigt sein. Beispielsweise kann das Zwischenelement 54 nicht ohne weiteres vom Zentralelement 52 gelöst werden. Auch das Außenelement 56 kann das Zwischenelement 54 derart umgeben bzw. umgreifen, dass ein Lösen nicht ohne eine Beschädigung ermöglicht ist. Zumindest zwei der drei Führungselemente 52, 54, 56, vorzugsweise alle drei Führungselemente 52, 54, 56, können integral gefertigt sein.

Fig. 19 zeigt eine abgewandelte Ausgestaltung einer Instrumentenhalterung 10, die mit einer Führungsvorrichtung 12 versehen ist, wobei die Führungsvorrichtung 12 grundsätzlich der Führungsvorrichtung, die anhand der Figuren 1 bis 17 beschrieben ist, ähnlich gestaltet ist. Dies trifft insbesondere auf die Ausgestaltung der Gelenkanordnung 50 zu. Auch die Führungsvorrichtung 12 gemäß Fig. 19 weist einen Führungsschaft 60 auf, der zur Aufnahme eines Schafts 16 eines Instruments 14 ausgebildet ist. Aus Veranschaulichungsgründen ist in Fig. 19 ferner ein mit 204 bezeichneter Sensor dargestellt, der dem ersten Antrieb 88 zugeordnet ist. Der Sensor 204 ist dazu ausgebildet, eine Schwenkbewegung bzw. eine Schwenkposition der Gelenkanordnung 50 bzw. des daran aufgenommenen Instruments 14 in Bezug auf die X-Achse zu erfassen. Beispielhaft kann der Sensor 204 der Übertragungsachse 132 und dem ersten Antrieb 88 zwischengeschaltet sein.

In bereits beschriebener Weise ist am Schaft 16 zumindest abschnittsweise eine zahnstangenartige Verzahnung 160 ausgebildet, in die ein Zahnrad 158 eingreift, das mit einem dritten Antrieb 92 gekoppelt ist. Der dritte Antrieb 92 kann auch als Translationsantrieb bezeichnet werden, vergleiche den Doppelpfeil 98 in Fig. 19.

Die anhand der Fig. 19 veranschaulichte Führungsvorrichtung 12 weist ferner einen vierten Antrieb 206 auf, der auch als Rotationsantrieb (Rotation um die Z-Achse) bezeichnet werden kann. Der Rotationsantrieb 206 ist an einem Antriebsgehäuse 208 aufgenommen, das der Führungseinheit 66 zugeordnet ist. Das Sensorgehäuse 88 und das Antriebsgehäuse 208 sind drehfest miteinander verbunden. Somit wird auch das Antriebsgehäuse 208 durch die Führungskette 80, die in Fig. 19 lediglich schematisch gebrochen dargestellt ist, an einer Verdrehung um die Z-Achse gehindert. In Bezug auf die Z-Achse ist daher der vierte Antrieb 206 gestellfest aufgenommen. Der vierte Antrieb 206 weist ein Ritzel 210 auf, das in ein Rad 212 eingreift, das am Führungsschaft 60 aufgenommen ist. Das Rad 212 ist verdrehfest mit dem Führungsschaft 60 gekoppelt. Mit anderen Worten bewirkt ein Antrieb des Rads 212 über das Ritzel 210 eine Verdrehung des Führungsschafts 60 um die Z-Achse. Gemeinsam mit dem Führungsschaft 60 werden der dritte Antrieb 92 und das im Führungsschaft 60 aufgenommene Instrument 14 um die Z-Achse verdreht.

Gemäß der anhand der Fig. 19 veranschaulichten Ausgestaltung weist die Führungsvorrichtung 12 für jede ihrer vier Bewegungsachsen (bzw. für jeden ihrer vier Freiheitsgrade) einen separaten Antrieb auf. Demgemäß kann für jeden der Bewegungsfreiheitsgrade eine Kraftrückwirkung (Force-Feedback) bzw. ein automatisierter Antrieb bereitgestellt werden. Dies kann weitere Anwendungsbereiche für die mit der Führungsvorrichtung 12 versehene Instrumentenhalterung 10 erschließen.

Fig. 20 zeigt eine schematisch stark vereinfachte Teilschnittansicht der sphärischen Gelenkanordnung 50. Wie vorstehend bereits veranschaulicht, weist die Gelenkanordnung 50 ein Zentralelement 52, ein Zwischenelement 54 und ein Außenelement 56 auf, die konzentrisch zueinander ausgerichtet sind. Das Außenelement 56 umschließt das Zwischenelement 54. Das Zwischenelement 54 umschließt das Zentralelement 52. Zwischen dem Zentralelement 52 und dem Zwischenelement 54 ist ein Kontaktbereich 220 ausgebildet. Zwischen dem Zwischenelement 54 und dem Außenelement 56 ist ein Kontaktbereich 222 ausgebildet.

Gemäß zumindest einigen Ausgestaltungen der vorliegenden Offenbarung werden das Zentralelement 52, das Zwischenelement 54 und das Außenelement 56 der Gelenkanordnung 50 gemeinsam integral durch ein generatives Fertigungsverfahren hergestellt. Demgemäß müssen fertigungsbedingt in den Kontaktbereichen 220, 222 bestimmte Abstände vorgehalten werden, da sich sonst schlichtweg keine separaten Teile gemeinsam fertigen lassen. Um jedoch im Betrieb eine hohe Führungsgenauigkeit (klapperfrei, geringes Spiel, usw.) gewährleisten zu können, sind die Kontaktbereiche 220, 222 zumindest abschnittsweise mit Erhebungen 216, 218, versehen, insbesondere mit Noppen.

Im Ausführungsbeispiel gemäß Fig. 20 ist die Kontaktfläche 140 des Zentralelements 52 mit ersten Erhebungen 216 versehen. Ferner ist die Kontaktfläche 144 des Zwischenelements 54 mit zweiten Erhebungen 218 versehen. Mit anderen Worten sind die äußeren Kontaktflächen 140, 144 des Zentralelements 52 bzw. des Zwischenelements 56 zumindest abschnittsweise mit Noppen versehen. Es versteht sich, dass die Erhebungen 216, 218 grundsätzlich auch den gegenüberliegenden Kontaktflächen 142, 146 ausgebildet sein können, die am Zwischenelement 54 bzw. am Außenelement 56 ausgebildet sind. Andere Kombinationen sind denkbar. Die Erhebungen 216, 218 erhöhen die Führungsgenauigkeit und verringern das Führungsspiel. Die Erhebungen 216, 218 werden gemeinsam mit den Führungselementen 52, 54, 56 integral generativ gefertigt. Die Erhebungen 216, 218 weisen nur äußerst geringe Kontaktflächen mit den benachbarten Führungselementen 54, 56 auf.

Beispielhaft können daher die Führungselemente 52, 54, 56 so gefertigt werden, dass die Erhebungen 216, 218 (im unmittelbar gefertigten Zustand) die benachbarten Führungselemente 54, 56 nur minimal oder gar nicht (stoffschlüssig) kontaktieren. Sollte es während der integralen gemeinsamen Fertigung tatsächlich zu einer Verbindung zwischen den Führungselementen 52, 54, 56 über deren Erhebungen 216, 218 kommen, so kann diese durch eine Relativbewegung zwischen einzelnen der Führungselemente 52, 54, 56 gelöst werden.

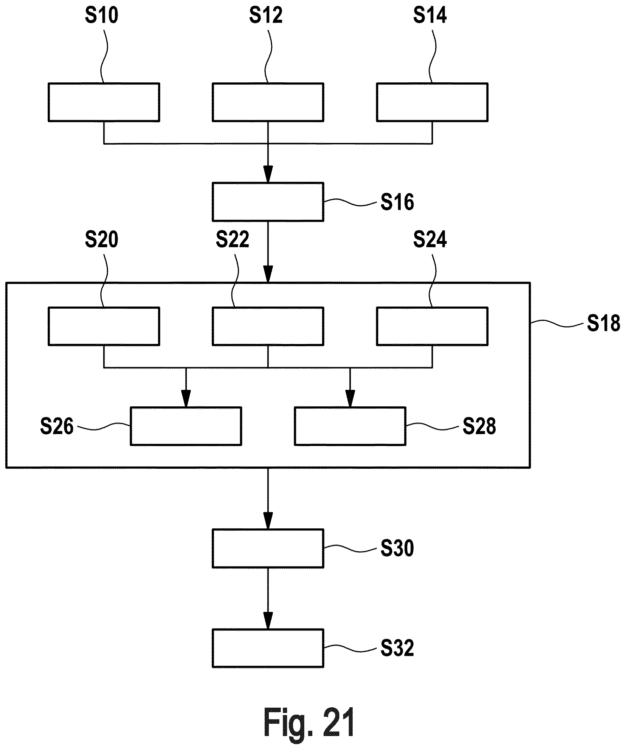

Mit Verweis auf Fig. 21 wird anhand eines schematischen Blockdiagramms eine Ausgestaltung eines Verfahrens zur Herstellung einer sphärischen Gelenkanordnung für eine Mehr-Achs-Führungsvorrichtung veranschaulicht.

Das Verfahren weist Schritte S10, S12 und S14 auf, die die Auslegung einer Gelenkanordnung mit ineinander verschachtelten, konzentrisch zueinander ausgerichteten Führungselementen umfassen. Der Schritt S10 umfasst die Auslegung eines Zentralelements. Der Schritt S12 umfasst die Auslegung eines Zwischenelements. Der Schritt S14 umfasst die Auslegung eines Außenelements. Es schließt sich ein Schritt S16 an, der die Bereitstellung eines (Computer-)Modells der Gelenkanordnung sowie die Übergabe an eine Vorrichtung zur generativen Fertigung umfasst.

In einem nachgelagerten Fertigungsschritt S18 wird die Gelenkanordnung generativ gefertigt. Der Schritt S18 umfasst (Teil-)Schritte S20, S22 und S24, die zumindest abschnittsweise gemeinsam durchgeführt werden. Der Schritt S20 betrifft die Fertigung des Zentralelements. Der Schritt S22 betrifft die Fertigung des Zwischenelements. Der Schritt S24 betrifft die Fertigung des Außenelements.

Der Schritt S18 umfasst ferner (Teil-)Schritte S26 und S28, die die Ausbildung von Kontaktbereichen zwischen den Führungselementen der Gelenkanordnung betreffen. Der Schritt S26 betrifft die Ausbildung eines Kontaktbereichs zwischen dem Zentralelement und dem Zwischenelement. Der Schritt S28 betrifft die Ausgestaltung eines Kontaktbereichs zwischen dem Zwischenelement und dem Außenelement. Vorzugsweise umfassen die Schritte S26 und S28 die Erzeugung einer Mehrzahl noppenartiger Erhebungen in den Kontaktbereichen zwischen dem Zentralelement, dem Zwischenelement und dem Außenelement. Somit können das Zentralelement, das Zwischenelement und das Außenelement einerseits hinreichend voneinander beabstandet sein, um die gemeinsame Fertigung der (separaten) Einzelelemente zu erlauben. Gleichwohl kann über die noppenartigen Erhebungen eine Reduktion des Spiels zwischen den Führungselementen bewirkt werden. Es versteht sich, dass die Schritte S26 und S28 zumindest abschnittsweise gleichzeitig zu den Schritten S20, S22 und S24 ablaufen.

An den Schritt S18 schließt sich ein Schritt S30 an, der eine Handhabung der unmittelbar gefertigten Gelenkanordnung umfasst. Sollten beispielsweise die in den Schritten S26 und S28 gefertigten noppenartigen Erhebungen das jeweils benachbarte Führungselement zumindest partiell kontaktieren, so kann beim Schritt S30 eine Trennung der Führungselemente voneinander erfolgen, um diese relativ zueinander verschwenkbar zu gestalten. Ferner kann der Schritt S30 eine Nachbearbeitung umfassen.

An den Schritt S30 schließt sich ein weiterer Schritt S32 an, der eine Anordnung und Ausrichtung der Gelenkanordnung in einer Führungsvorrichtung umfasst. Zu diesem Zweck werden die Führungselemente, zumindest das Zwischenelement und das Außenelement in eine definierte Betriebsorientierung bewegt. Auf diese Weise können etwa Führungsausnehmungen, die in das Zwischenelement und das Außenelement eingebracht sind, in eine definierte Relativausrichtung gebracht werden. Ferner kann das Zentralelement mit einem Führungsschaft gekoppelt werden, der die Führungsausnehmungen des Zwischenelements und des Außenelements zumindest teilweise durchragt. Auf diese Weise wird die Gelenkanordnung insgesamt in die gewünschte Betriebskonfiguration überführt, in der die Gelenkanordnung ähnlich einer kardanischen Lagerung zwei definierte Schwenkachsen für den am Zentralelement aufgenommenen Führungsschaft bzw. für ein im Führungsschaft aufgenommenes Instrument bereitstellt.

Die Gelenkanordnung mitsamt der Führungsausnehmungen, weiterer Durchbrüche sowie der noppenartigen Erhebungen zwischen den einzelnen Führungselementen kann in einfacher Weise integral einstückig generativ gefertigt werden. Auf diese Weise kann der Fertigungsaufwand deutlich minimiert werden. Insbesondere sinkt der Montageaufwand, da die Führungselemente in gewisser Weise bereits miteinander gefügt sind, wenn die generative Fertigung beendet ist.

## Patentansprüche

1. Gelenkanordnung (50) für eine Mehr-Achs-Führungsvorrichtung (12) für ein Instrument (14), wobei die Gelenkanordnung (50) eine Mehrzahl von konzentrisch zueinander ausgerichteten, zumindest abschnittweise sphärisch gestalteten Führungselementen (52, 54, 56) aufweist, die als Bestandteil einer kombinierten sphärischen Lagerung (58) ausgebildet sind und sphärische Kontaktbereiche (220, 222) umfassen, **dadurch gekennzeichnet, dass** zumindest zwei der Führungselemente (52, 54, 56) generativ und integral in konzentrischer Ausrichtung gefertigt sind, und wobei die zumindest zwei Führungselemente (52, 54, 56) eine Relativverschwenkung erlauben.

2. Gelenkanordnung (50) nach Anspruch 1, wobei an den zumindest zwei Führungselemente (52, 54, 56) zumindest eine mit einer Mehrzahl von Erhebungen (216) versehene Kontaktfläche (140, 142, 144, 146, 148) ausgebildet ist, insbesondere eine genoppte Kontaktfläche (140, 142, 144, 146, 148).

3. Gelenkanordnung (50) nach Anspruch 1 oder 2, umfassend drei Führungselemente (52, 54, 56), die ein Zentralelement (52), ein Zwischenelement (54) und ein Außenelement (56) ausbilden, die zueinander konzentrisch angeordnet und integral in konzentrischer Ausrichtung gefertigt sind.

4. Gelenkanordnung (50) nach Anspruch 3, wobei das Außenelement (56) als Kugelschale gestaltet ist und das Zwischenelement (54) verliersicher umschließt, wobei das Zwischenelement (54) als Kugelschale gestaltet ist und das Zentralelement (52) verliersicher umschließt, und wobei das Zentralelement (52) vorzugsweise eine mittige, durchgehende Führungsöffnung (154) aufweist, die zur Einführung eines Instrumentenschaftes (16) oder eines Führungsschaftes (60) ausgebildet ist.

5. Gelenkanordnung (50) nach Anspruch 3 oder 4, wobei das Zwischenelement (54) und das Außenelement (56) Führungsausnehmungen (104, 106) aufweisen, vorzugsweise nutartige Führungsausnehmungen (104, 106), wobei zumindest eine Führungsausnehmung (104) des Zwischenelements (54) und zumindest eine Führungsausnehmung (106) des Außenelements (56) zueinander winklig versetzt sind, insbesondere um 90° zueinander versetzt ausgerichtet, und wobei das Zentralelement (52) vorzugsweise mit einem Führungsschaft (60) versehen oder koppelbar ist, der die nutartigen Führungsausnehmungen (104, 106) des Zwischenelements (54) und des Außenelements (56) zumindest teilweise durchragt.

6. Gelenkanordnung (50) nach Anspruch 5, wobei am Außenelement (56) ferner zumindest eine nutartige Durchführung (108) für eine Übertragungsachse (132) ausgebildet ist, die mit dem Zwischenelement (54) koppelbar ist, wobei die Führungsausnehmung (104) und die Durchführung (108) zueinander winklig versetzt und insbesondere um 90° zueinander versetzt ausgerichtet sind.

7. Mehr-Achs-Führungsvorrichtung (12) für ein Instrument (14), insbesondere ein medizinisches Instrument oder ein Simulationsinstrument, mit einem Basisgestell (30), und einer Gelenkanordnung (50) nach einem der vorhergehenden Ansprüche, die am Basisgestell (30) aufgenommen ist, wobei die Gelenkanordnung (50) zwei nichtparallele Schwenkachsen (X, Y) bereitstellt, die sich vorzugsweise in einem Zentrum der Gelenkanordnung (50) schneiden.

8. Führungsvorrichtung (12) nach Anspruch 7, ferner aufweisend einen ersten Schwenkaktuator (88) für die erste Schwenkachse (X), der mit einem Zwischenelement (54) der Gelenkanordnung (50) gekoppelt ist, einen zweiten Schwenkaktuator (90) für die zweite Schwenkachse (Y), der mit einem Außenelement (56) der Gelenkanordnung (50) gekoppelt ist, wobei der erste Schwenkaktuator (88) über eine Übertragungsachse (132) mit dem Zwischenelement (54) gekoppelt ist, die das Außenelement (56) durchragt.

9. Führungsvorrichtung (12) nach Anspruch 7 oder 8, ferner aufweisend einen Führungsschaft (60), der an ein Zentralelement (52) der Gelenkanordnung (50) gekoppelt ist und eine Geradführung für das Instrument (14) ausbildet, wobei der Führungsschaft (60) und das Zentralelement (52) vorzugsweise eine Rotationsachse (Z) für das Instrument (14) definieren.

10. Führungsvorrichtung (12) nach Anspruch 9, ferner aufweisend zumindest einen Translationsaktuator (98) oder einen Rotationsaktuator (206), wobei der Translationsaktuator (98) dazu ausgebildet ist, zur translatorischen Kraftübertragung an einen Instrumentenschaft (16) des Instruments (14) anzugreifen, der in den Führungsschaft (60) eingeführt ist, und wobei der Rotationsaktuator (206) dem Führungsschaft (60) zugeordnet und dazu ausgebildet ist, zur rotatorischen Kraftübertragung mittelbar oder unmittelbar an den Instrumentenschaft (16) anzugreifen, der in den Führungsschaft (60) eingeführt ist.

11. Führungsvorrichtung (12) nach Anspruch 9 oder 10, wobei der Führungsschaft (60) über eine Führungseinheit (66) mit dem Basisgestell (30) gekoppelt ist, und wobei die Führungseinheit (66) mit einer Verdrehsicherung (80) gekoppelt ist, vorzugsweise mit einer Führungskette, die das Basisgestell (30) und die Führungseinheit (66) gelenkig miteinander verbindet.

12. Führungsvorrichtung (12) nach einem der Ansprüche 9 bis 11, ferner aufweisend eine Sensoreinheit (70), die dem Führungsschaft (60) zugeordnet ist, wobei die Sensoreinheit (70) dazu ausgebildet ist, eine Translationsbewegung und eine Rotationsbewegung eines eingeführten Instruments (14) zu erfassen, und wobei die Sensoreinheit (70) einen mehrdimensional wirksamen Sensor oder zwei miteinander gekoppelte Sensoren aufweist, die gemeinsam zur Erfassung der Translationsbewegung und der Rotationsbewegung ausgebildet sind.

13. Verfahren zur Herstellung einer Gelenkanordnung (50) für eine Mehr-Achs-Führungsvorrichtung (12) für ein Instrument (14), das Folgendes umfasst:
generative Fertigung einer Mehrzahl von zumindest abschnittweise sphärisch gestalteten Führungselementen (52, 54, 56) mit sphärischen Kontaktbereichen (220, 222) zur Erzeugung einer kombinierten sphärischen Lagerung (58),
wobei zumindest zwei der Führungselemente (52, 54, 56) integral in konzentrischer Ausrichtung gefertigt werden, und wobei die zumindest zwei Führungselemente (52, 54, 56) eine Relativverschwenkung erlauben, und
vorzugsweise Erzeugen zumindest einer mit einer Mehrzahl von Erhebungen (216) versehenen Kontaktflächen (140, 142, 144, 146, 148), insbesondere einer genoppten Kontaktfläche, in den Kontaktbereichen (220, 222).

14. Verfahren nach Anspruch 13, ferner umfassend:
generative, integrale Fertigung eines Zentralelements (52), eines Zwischenelements (54) und eines Außenelements (56), die drei Führungselemente (52, 54, 56) bilden, die zueinander konzentrisch angeordnet sind,
wobei das Außenelement (56) als Kugelschale gestaltet wird und das Zwischenelement (54) verliersicher umschließt,
wobei das Zwischenelement (54) als Kugelschale gestaltet wird und das Zentralelement (52) verliersicher umschließt, und
wobei das Zentralelement (52) eine mittige, durchgehende Führungsöffnung (154) aufweist, die zur Einführung eines Instrumentenschaftes (16) oder eines Führungsschaftes (60) ausgebildet ist.

15. Verwendung einer Gelenkanordnung (50), die gemäß den Ansprüchen 1 bis 6 gestaltet und/oder gemäß den Ansprüchen 13 oder 14 gefertigt ist, als sphärische Lagerung bei einer Instrumentenhalterung (10), insbesondere einer Instrumentenhalterung für ein Simulationssystem oder ein Assistenzsystem für minimal-invasive Eingriffe.

## Claims

1. A joint assembly (50) for a multi-axis guiding device (12) for an instrument (14), wherein the joint assembly (50) comprises a plurality of guiding elements (52, 54, 56) that are aligned concentrically with respect to one another and that are at least sectionally spherically arranged, wherein the guiding elements (52, 54, 56) are configured as a component of a combined spherical bearing (58) and comprise spherical contact areas (220, 222), **characterized in that** at least two of the guiding elements (52, 54, 56) are additively and integrally manufactured in a concentric alignment, and wherein the at least two guiding elements (52, 54, 56) enable a relative pivot movement.

2. The joint assembly (50) according to claim 1, wherein at least one guide surface (140, 142, 144, 146, 148) that is provided with a plurality of protrusions (216), particularly a nubby contact surface (140, 142, 144, 146, 148), is formed at the at least two guiding elements (52, 54, 56).

3. The joint assembly (50) according to claim 1 or 2, comprising three guiding elements (52, 54, 56) that form a central element (52), an intermediate element (54) and an outer element (56), and that are arranged concentrically with respect to one another and integrally manufactured in a concentric alignment.

4. The joint assembly (50) according to claim 3, wherein the outer element (56) is arranged as a spherical shell and undetachably surrounds the intermediate element (54), wherein the intermediate element (54) is arranged as a spherical shell and undetachably surrounds the central element (52), and wherein the central element (52) preferably comprises a central guide opening (154) extending therethrough that is arranged for the insertion of an instrument shaft (16) or a guiding shaft (60).

5. The joint assembly (50) according to claim 3 or 4, wherein the intermediate element (54) and the outer element (56) comprise guiding recesses (104, 106), preferably groove-shaped guiding recesses (104, 106), wherein at least one guiding recess (104) of the intermediate element (54) and at least one guiding recess (106) of the outer element (56) are angularly offset from one another and particularly oriented at an offset of 90° from one another, and wherein the central element (52) is preferably provided with or arranged to be coupled to a guiding shaft (60) that at least partially extends through the groove-shaped guiding recesses (104, 106) of the intermediate element (54) and the outer element (56).

6. The joint assembly (50) according to claim 5, wherein at the outer element (56) at least one groove-shaped passage (108) for a transmission axis (132) is provided, wherein the passage (108) is arranged to be coupled with the intermediate element (54), wherein the guiding recess (104) and the passage (108) are angularly offset from one another and particularly oriented at an offset of 90° from one another.

7. A multi-axis guiding device (12) for an instrument (14), particularly a medical instrument or a simulation instrument, comprising a base frame (30) and a joint assembly (50) according to any one of the preceding claims, wherein the joint assembly (50) is mounted to the base frame (30), wherein the joint assembly (50) provides two non-parallel pivot axes (X, Y) that preferably intersect in a center of the joint assembly.

8. The guiding device (12) according to claim 7, further comprising a first pivot actuator (88) for a first pivot axis (X) that is coupled to the intermediate element (54) of the joint assembly (50), a second pivot actuator (90) for the second pivot axis (Y) that is coupled to the outer element (56) of the joint assembly (50), wherein the first pivot actuator (88) is coupled via a transmission axis (132) to the intermediate element (54), wherein the transmission axis (132) extends through the outer element (56).

9. The guiding device (12) according to claim 7 or 8, further comprising a guiding shaft (60) that is coupled to a central element (52) of the joint assembly (50) and that forms a linear guide for the instrument (14), wherein the guiding shaft (60) and the central element (52) preferably further define a rotation axis (Z) for the instrument (14).

10. The guiding device (12) according to claim 9, further comprising a translational actuator (98) or a rotational actuator (206), a wherein the translational actuator (98) is configured to engage an instrument shaft (16) of the instrument (14), which is inserted in the guiding shaft (60), for translational force transmission, and wherein the rotational actuator (206) is assigned to the guiding shaft (60) and configured to mediately or directly engage the instrument shaft (16), which is inserted in the guiding shaft (60), for rotatory force transmission.

11. The guiding device (12) according to any one of claims 9 or 10, wherein der guiding shaft (60) is coupled via a guiding unit (66) with the base frame (30), wherein the guiding unit (66) is coupled with a rotation prevention feature (80), preferably with a guiding chain that forms a hinged connection between the base frame (30) and the guiding unit (66).

12. The guiding device (12) according to any one of claims 9 to 11, further comprising a sensor unit (70), that is assigned to the guiding shaft (60), wherein the sensor unit (70) is arranged to detect a translational movement and a rotation movement of an inserted instrument (14), and wherein the sensor unit (70) comprises a sensor that is multi-dimensionally operative, or two sensors that are coupled to one another and that are jointly configured for detecting the translational movement and the rotation movement.

13. A method of manufacturing a joint assembly (50) for a multi-axis guiding device (12) for an instrument (14), comprising the following steps:
additive manufacturing of a plurality of guiding elements (52, 54, 56) that are at least sectionally spherically formed, and that are provided with spherical contact areas (220, 222) for forming a combined spherical bearing (58),
wherein at least two of the guiding elements (52, 54, 56) are integrally manufactured in a concentric alignment, and wherein the at least two guiding elements (52, 54, 56) enable a relative pivot movement, and
preferably forming at least one contact surface (140, 142, 144, 146, 148) that is provided with a plurality of protrusions (216), particularly a nubby contact surface, in the contact areas (220, 222).

14. The method according to claim 13, further comprising:
additive, integral manufacturing of a central element (52), an intermediate element (54) and an outer element (56), that form three guiding elements (52, 54, 56) that are concentrically arranged with respect to one another, wherein the outer element (56) is arranged as a spherical shell and undetachably surrounds the intermediate element (54),
wherein the intermediate element (54) is arranged as a spherical shell and undetachably surrounds the central element (52), and
wherein the central element comprises a central guide opening (154) extending therethrough that is configured for inserting an instrument shaft (16) or a guiding shaft (60).

15. A use of a joint assembly (50) that is arranged in accordance with the claims 1 to 6 and/or that is manufactured in accordance with the claims 13 or 14 as a spherical bearing in an instrument holder (10), particularly an instrument holder for a simulation system or an assistance system for minimally-invasive operations.

## Revendications

1. Ensemble articulé (50) pour un dispositif de guidage multiaxe (12) pour un instrument (14), dans lequel l'ensemble articulé (50) présente une multiplicité d'éléments de guidage (52, 54, 56) au moins en partie de forme sphérique, orientés de façon concentrique l'un par rapport à l'autre, qui sont réalisés sous forme de composants d'un palier sphérique combiné (58) et qui comprennent des zones de contact sphériques (220, 222), **caractérisé en ce qu'**au moins deux des éléments de guidage (52, 54, 56) sont fabriqués de façon générative et intégralement en orientation concentrique, et dans lequel lesdits au moins deux éléments de guidage (52, 54, 56) permettent un pivotement relatif.

2. Ensemble articulé (50) selon la revendication 1, dans lequel au moins une face de contact (140, 142, 144, 146, 148) garnie d'une multiplicité de protubérances (216) est formée sur lesdits au moins deux éléments de guidage (52, 54, 56), en particulier une face de contact dotée de reliefs (140, 142, 144, 146, 148).

3. Ensemble articulé (50) selon une revendication 1 ou 2 comprenant trois éléments de guidage (52, 54, 56), qui forment un élément central (52), un élément intermédiaire (54) et un élément extérieur (56), qui sont disposés de façon concentrique l'un par rapport à l'autre et qui sont fabriqués intégralement en orientation concentrique.

4. Ensemble articulé (50) selon la revendication 3, dans lequel l'élément extérieur (56) est réalisé en forme de coquille sphérique et entoure l'élément intermédiaire (54) de façon imperdable, dans lequel l'élément intermédiaire (54) est réalisé en forme de coquille sphérique et entoure l'élément central (52) de façon imperdable, et dans lequel l'élément central (52) présente de préférence une ouverture de guidage centrale continue (154), qui est configurée en vue de l'introduction d'une tige d'instrument (16) ou d'une tige de guidage (60).

5. Ensemble articulé (50) selon une revendication 3 ou 4, dans lequel l'élément intermédiaire (54) et l'élément extérieur (56) présentent des évidements de guidage (104, 106), de préférence des évidements de guidage en forme de rainures (104, 106), dans lequel au moins un évidement de guidage (104) de l'élément intermédiaire (54) et au moins un évidement de guidage (106) de l'élément extérieur (56) sont angulairement décalés l'un par rapport à l'autre, en particulier sont orientés à 90° l'un de l'autre, et dans lequel l'élément central (52) est de préférence muni d'une tige de guidage (60) ou peut être couplé à une tige de guidage (60), qui traverse au moins en partie les évidements de guidage en forme de rainures (104, 106) de l'élément intermédiaire (54) et de l'élément extérieur (56).

6. Ensemble articulé (50) selon la revendication 5, dans lequel au moins un passage en forme de rainure (108) est en outre formé sur l'élément extérieur (56) pour un axe de transmission (132), qui peut être couplé à l'élément intermédiaire (54), dans lequel l'évidement de guidage (104) et le passage (108) sont angulairement décalés l'un par rapport à l'autre, et sont en particulier orientés avec un décalage de 90° l'un par rapport à l'autre.

7. Dispositif de guidage multiaxe (12) pour un instrument (14), en particulier un instrument médical ou un instrument de simulation, avec une structure de base (30) et un ensemble articulé (50) selon l'une quelconque des revendications précédentes, qui est logé sur la structure de base (30), dans lequel l'ensemble articulé (50) procure deux axes de pivotement non parallèles (X, Y), qui se coupent de préférence en un centre de l'ensemble articulé (50).

8. Dispositif de guidage (12) selon la revendication 7, présentant en outre un premier actionneur de pivotement (88) pour le premier axe de pivotement (X), qui est couplé à un élément intermédiaire (54) de l'ensemble articulé (50), un deuxième actionneur de pivotement (90) pour le deuxième axe de pivotement (Y), qui est couplé à un élément extérieur (56) de l'ensemble articulé (50), dans lequel le premier actionneur de pivotement (88) est couplé à l'élément intermédiaire (54) par un axe de transmission (132), qui traverse l'élément extérieur (56).

9. Dispositif de guidage (1) selon une revendication 7 ou 8, présentant en outre une tige de guidage (60), qui est couplée à un élément central (52) de l'ensemble articulé (50) et forme un guidage droit pour l'instrument (14), dans lequel la tige de guidage (60) et l'élément central (52) définissent de préférence un axe de rotation (Z) pour l'instrument (14).

10. Dispositif de guidage (12) selon la revendication 9, présentant en outre un actionneur de translation (98) ou un actionneur de rotation (206), dans lequel l'actionneur de translation (98) est configuré pour agir pour la transmission de force par translation à une tige d'instrument (16) de l'instrument (14), qui est introduite dans la tige de guidage (60), et dans lequel l'actionneur de rotation (206) est associé à la tige de guidage (60) et est configuré pour agir pour la transmission de force par rotation indirectement ou directement à la tige d'instrument (16), qui est introduite dans la tige de guidage (60).

11. Dispositif de guidage (12) selon une revendication 9 ou 10, dans lequel la tige de guidage (60) est couplée à la structure de base (30) par une unité de guidage (66), et dans lequel l'unité de guidage (66) est couplée à un blocage de rotation (80), de préférence à une chaîne de guidage, qui relie la structure de base (30) et l'unité de guidage (66) l'une à l'autre de façon articulée.

12. Dispositif de guidage (12) selon l'une quelconque des revendications 9 à 11, présentant en outre une unité de capteurs (70), qui est associée à la tige de guidage (60), dans lequel l'unité de capteurs (70) est configurée pour détecter un mouvement de translation et un mouvement de rotation d'un instrument introduit (14), et dans lequel l'unité de capteurs (70) présente un capteur à action multidimensionnelle ou deux capteurs couplés l'un à l'autre, qui sont configurés en commun pour la détection du mouvement de translation et du mouvement de rotation.

13. Procédé de production d'un ensemble articulé (50) pour un dispositif de guidage multiaxe (12) pour un instrument (14), qui comprend les opérations suivantes:
fabrication générative d'une multiplicité d'éléments de guidage de forme au moins en partie sphérique (52, 54, 56) avec des zones de contact sphériques (220, 222) pour la production d'un palier sphérique combiné (58),
dans lequel on fabrique au moins deux des éléments de guidage (52, 54, 56) intégralement en orientation concentrique, et dans lequel lesdits au moins deux éléments de guidage (52, 54, 56) permettent un pivotement relatif, et
de préférence production d'au moins une face de contact (140, 142, 144, 146, 148) garnie d'une multiplicité de protubérances (216), en particulier d'une face de contact dotée de reliefs, dans les zones de contact (220, 222).

14. Procédé selon la revendication 13, comprenant en outre:
la production générative intégrale d'un élément central (52), d'un élément intermédiaire (54) et d'un élément extérieur (56), qui forment trois éléments de guidage (52, 54, 56), qui sont disposés de façon concentrique l'un par rapport à l'autre,
dans lequel l'élément extérieur (56) est réalisé en forme de coquille sphérique et entoure l'élément intermédiaire (54) de façon imperdable,
dans lequel l'élément intermédiaire (54) est réalisé en forme de coquille sphérique et entoure l'élément central (52) de façon imperdable, et
dans lequel l'élément central (52) présente une ouverture de guidage centrale continue (154), qui est configurée en vue de l'introduction d'une tige d'instrument (16) ou d'une tige de guidage (60).

15. Utilisation d'un ensemble articulé (50), qui est configuré selon les revendications 1 à 6 et/ou qui est fabriqué selon les revendications 13 ou 14, sous forme de palier sphérique dans un support d'instrument (10), en particulier dans un support d'instrument pour un système de simulation ou un système d'assistance pour des interventions à invasion minimale.
